# EUROPEAN PATENT SPECIFICATION

(11) **EP 3 081 943 B1**
(45) Date of publication and mention of the grant of the patent: **07.11.2018**
(21) Application number: 16164532.0
(22) Date of filing: 08.04.2016
(51) Int. Cl.: G01N 35/10

(54) **BIOLOGICAL SAMPLE MEASUREMENT DEVICE, BIOLOGICAL SAMPLE MEASUREMENT SYSTEM, AND BIOLOGICAL SAMPLE MEASUREMENT METHOD**
BIOLOGISCHE PROBENMESSVORRICHTUNG, BIOLOGISCHES PROBENMESSSYSTEM UND BIOLOGISCHES PROBENMESSVERFAHREN
DISPOSITIF, SYSTÈME ET PROCÉDÉ DE MESURE D'ÉCHANTILLON BIOLOGIQUE

(30) Priority: 14.04.2015 JP 2015082192
(43) Date of publication of application: 19.10.2016
(73) Proprietor: ARKRAY, Inc., Minami-ku Kyoto-shi, Kyoto 601-8045 (JP)
(72) Inventor: NAKAJIMA, Shinya, Kyoto-shi, Kyoto 602-0008 (JP)
(74) Representative: MacDougall, Alan John Shaw

(56) References cited:
- EP-A1- 2 799 887
- EP-A1- 2 878 956
- EP-B1- 0 720 747
- JP-A- H10 293 132
- US-A- 6 063 340

## Description

The present invention relates to a washing technique of a portion contacted by a biological sample in a biological sample measurement device or a biological sample measurement system.

In a biological sample measurement device, when the concentration of a component contained in one biological sample is high, there are cases where the measurement result of the next biological sample is affected with a washing method in which a flow path of the biological sample and the like are uniformly washed with the same condition. In order to solve this problem, conventionally, the way of washing a portion contacted by the biological sample is changed based on the measurement result of a predetermined component contained in the biological sample.

For example, Japanese Patent Application Laid-open No. H10-293132 describes a measurement method of a biological sample that performs additional washing before measurement of the next biological sample in the case where the concentration of a component contained in the biological sample is high. Specifically, it is judged that the concentration of glucose in one biological sample is high from a relationship with measurement time, and necessity for the additional washing is determined for the measurement of the next biological sample.

However, the above related art has had the following problem.

That is, in Japanese Patent Application Laid-open No. H10-293132, it is determined whether or not the additional washing is performed after the measurement of one biological sample is completed. As a result, there has been a problem that the throughput of a device is reduced with this method.

EP 2799887 discloses an automatic analyzer capable of suppressing carry-over from a sample of one type to a sample of a different type of lower concentration with a requisite minimum of washing operation and thereby improving the accuracy of the results of measurements. When the type is to be changed from serum (preceding sample) to urine (current sample), "serum" is set to a preceding type and "urine" is set to a measurement type at number 1 in a condition number. At condition number 1, the wash type is pattern 1, with washing performed once with detergent 1. Where the preceding sample is serum and the current sample is CSF, the condition number is 2 and the wash type is pattern 2, with washing performed twice using detergent 1 and once with detergent 2. Where the preceding sample is urine and the current sample is CSF, the condition number is 3 and the wash type is pattern 3, with washing performed once with detergent 1, once with detergent 2, and once with water. In the case of pattern 4, washing is performed three times with detergent 1.

EP 0720747 discloses an automated, continuous and random access analytical system, having apparatus and methodology capable of simultaneously performing multiple assays of liquid samples using different assay methodologies, and providing continuous and random access while performing a plurality of different assays on the same or different samples during the same time period. A method is also disclosed of operating an automated continuous and random access analytical system capable of simultaneously effecting multiple assays of a plurality of liquid samples wherein scheduling of various assays of the plurality of liquid samples is followed by creating a unit dose disposable and separately transferring a first liquid sample and reagents to a reaction vessel without initiation of an assay reaction sequence, followed by physical transfer of the unit dose disposable to a processing workstation, whereby a mixture of the unit dose disposable reagents and sample are achieved during incubation. The system is capable of performing more than one scheduled assay in any order, and assays where more than such scheduled assays are presented. The automated, continuous and random access analytical system is also capable of analyzing the incubated reaction mixtures independently and individually by at least two assay procedures.

EP2878956 discloses an automated analyzer that can reduce contamination of a diluted low-concentration specimen resulting from a high-concentration specimen not being diluted. The automated analyzer includes a specimen nozzle that performs both the function of pipetting a specimen from a specimen container accommodating the specimen and the function of pipetting a specimen diluted by the analyzer, and means for washing the specimen nozzle with a predetermined detergent. When a pipetting process of a high-concentration specimen not being diluted and a pipetting process of a low-concentration specimen diluted by the analyzer are consecutively performed for the same specimen by the specimen nozzle, between the pipetting process of a high-concentration specimen and the pipetting process of a low-concentration specimen, the analyzer performs a washing processing in which the specimen nozzle is washed with the predetermined detergent.

The present invention is defined in the appended independent claims.

The present invention has been devised in view of the above circumstances, and an object of at least some embodiments is to provide a biological sample measurement device, a biological sample measurement system, and a biological sample measurement method each having a high throughput obtained by being capable of early determining whether or not change of a washing condition is necessary.

In order to alleviate the above problem, embodiments of the present invention adopt the following technical means.

A biological sample measurement device provided by a first aspect of the present invention is a biological sample measurement system comprising: a biological sample measurement device and external equipment connected to the biological sample measurement device, wherein the biological sample measurement device comprises: a measurement section for performing measurement of a biological sample; a washing section for washing a biological sample contact portion that contacts the biological sample during the measurement; a characteristic judgment section for judging a characteristic of the biological sample before the measurement is completed; and a washing control section for determining a washing method by the washing section based on the characteristic judged by the characteristic judgment section, the characteristic judgment section is configured to acquire the characteristic from the external equipment, the external equipment is a urine qualitative measurement device that is configured to perform urine qualitative measurement on the urine by using a urine test paper to acquire a urine qualitative measurement result of a predetermined measurement item, the biological sample is urine, the biological sample measurement device is configured to perform measurement on the urine on which the urine qualitative measurement has been performed in the urine qualitative measurement device, the characteristic judgment section is configured to receive the urine qualitative measurement result from the urine qualitative measurement device and to determine a level of the urine qualitative measurement result as the characteristic, the level of the urine qualitative measurement result is a level of urine occult blood, and the washing control section is configured to determine a washing method of the biological sample contact portion contacted by the urine used for the urine qualitative measurement by the washing section based on the level.

Preferably, the measurement section is further configured to perform measurement of a plurality of types of biological samples sequentially, urine being one of the plurality of types.

Preferably, the characteristic judgment section is further configured to determine the type of the biological sample as a further characteristic, and the washing control section is further configured to determine the washing method based on the type.

Preferably, the type is one of urine and feces, and the characteristic judgment section is further configured to determine whether the type is the urine or the feces as the further characteristic.

Preferably, in a case where the measurement section performs the measurement of the feces subsequently to the measurement of the urine, the washing control section is further configured to cause the washing section to wash the biological sample contact portion contacted by the urine under a condition in which a washing liquid quantity or washing time is increased.

Preferably, the biological sample measurement device further includes a measurement control section that controls a measurement method in the measurement section, the measurement section measures an occult blood, and the measurement control section uses a common measurement reagent irrespective of the type.

Preferably, the biological sample is contained in a sample container on which subject identification information is recorded, the biological sample measurement device further includes a reading section that reads the subject identification information, and a storage section that stores in advance pair information in which the subject identification information is paired with the type associated with the subject identification information, and the characteristic judgment section is further configured to refer to the pair information based on the subject identification information read by the reading section to determine the type as the further characteristic.

Preferably, the storage section stores a measurement schedule table in which a schedule of the measurement of the biological sample is described, the measurement schedule table includes the pair information, and, in a case where, subsequently to the measurement of a first biological sample of a predetermined type, the measurement of a second biological sample of a predetermined type different from the first biological sample is scheduled to be performed in the measurement schedule table, the washing control section is further configured to determine the washing method of the biological sample contact portion contacted by the first biological sample in accordance with a combination of the first biological sample and the second biological sample.

Preferably, the biological sample is contained in a sample container on which subject identification information is recorded, the biological sample measurement device further includes a reading section that reads the subject identification information, the type is also recorded in the sample container, the reading section is capable of reading the type, and the characteristic judgment section is further configured to determine the type of the biological sample read by the reading section as the characteristic.

Preferably, in a case where, subsequently to the measurement of a first biological sample of a predetermined type, the measurement of a second biological sample of a predetermined type different from the first biological sample is scheduled to be performed, the washing control section is further configured to determine the washing method of the biological sample contact portion contacted by the first biological sample in accordance with a combination of the first biological sample and the second biological sample.

Preferably, the predetermined measurement item is an occult blood, the characteristic judgment section determines the urine qualitative measurement result as the characteristic, and, in a case where the characteristic judgment section determines that the urine qualitative measurement result of the occult blood is not less than a preset first threshold value, the washing control section causes the washing section to wash the biological sample contact portion with a condition in which a washing liquid quantity or washing time is increased.

Preferably, the biological sample measurement system further includes a measurement control section that controls a measurement method in the measurement section, the predetermined measurement item is an occult blood, the measurement section is capable of further performing urine occult blood quantitative measurement on the urine on which the urine qualitative measurement of the occult blood has been performed in the urine qualitative measurement device, and the measurement control section causes the measurement section to perform the urine occult blood quantitative measurement in a case where the urine qualitative measurement result of the occult blood is not less than a preset second threshold value.

A biological sample measurement method provided by a second aspect of the present invention is a biological sample measurement method, comprising: a measurement step of performing measurement of a biological sample; a washing step of washing a biological sample contact portion, the biological sample contact portion contacting the biological sample during the measurement; a urine qualitative measurement step of performing urine qualitative measurement on the urine by using a urine test paper to acquire a urine qualitative measurement result of a predetermined measurement item before the measurement step is completed; a characteristic judgment step of judging a characteristic of the biological sample before the measurement is completed; and a determination step of determining a washing method of the biological sample contact portion based on the characteristic judged in the judgment step; wherein the biological sample is urine, in the measurement step, the measurement is performed on the urine on which the urine qualitative measurement has been performed in the urine qualitative measurement step, in the characteristic judgment step, a level of the urine qualitative measurement result is judged as the characteristic, the level of the urine qualitative measurement result is a level of urine occult blood, and in the determination step, a washing method of the biological sample contact portion contacted by the urine used for the urine qualitative measurement is determined based on the level of the urine qualitative measurement result.

Preferably, the measurement of a plurality of types of biological samples can be performed in the measurement step, the type of the biological sample is determined as the characteristic in the judgment step, and the washing method is determined based on the type in the determination step.

According to the adoption of the aspect of the present invention, it is possible to provide the biological sample measurement system, and the biological sample measurement method each having the high throughput obtained by being capable of early determining whether or not the change of the washing condition is necessary.

Other features and advantages of the present invention will become more apparent from the description of exemplary embodiments made below with reference to the accompanying drawings.
FIG. 1 is a schematic perspective view showing a biological sample measurement device according to a first exemplary system;
FIG. 2 is a block diagram of the biological sample measurement device shown in FIG. 1;
FIG. 3 is a perspective view of a principal portion showing an internal configuration of the biological sample measurement device shown in FIG. 1;
FIG. 4 is a schematic configuration diagram of a washing section of the biological sample measurement device shown in FIG. 1;
FIG. 5A is a table showing an example of a measurement schedule table stored in the biological sample measurement device shown in FIG. 1, and FIG. 5B is a table showing an example of a washing schedule table stored in the biological sample measurement device shown in FIG. 1;
FIG. 6 is a flowchart showing an example of operation process procedures of a control section of the biological sample measurement device shown in FIG. 1;
FIG. 7 is a flowchart showing an example of a washing method determination subroutine of a biological sample flow path in the flowchart shown in FIG. 6;
FIG. 8 is a schematic perspective view showing a biological sample measurement system according to a first embodiment of the present invention;
FIG. 9 is a block diagram of the biological sample measurement system shown in FIG. 8;
FIG. 10 is a perspective view of a principal portion showing an internal configuration of a urine qualitative measurement device constituting the biological sample measurement system shown in FIG. 8;
FIG. 11 is a table showing an example of a measurement item comparison table stored in the biological sample measurement system shown in FIG. 8;
FIG. 12 is a table showing an example of a urine quantitative measurement necessity determination table stored in the biological sample measurement system shown in FIG. 8;
FIG. 13 is a table showing an example of an additional washing necessity determination table stored in the biological sample measurement system shown in FIG. 8;
FIG. 14A is a table showing an example of a quantitative measurement schedule table stored in the biological sample measurement system shown in FIG. 8, and FIG. 14B is a table showing an example of a washing schedule table stored in the biological sample measurement system shown in FIG. 8;
FIG. 15A is a measurement range comparison table of measurement items common to the urine qualitative measurement device and the biological sample measurement device constituting the biological sample measurement system shown in FIG. 8, and FIG. 15B is a table showing an example of a quantitative measurement item classification table stored in the biological sample measurement system shown in FIG. 8;
FIG. 16 is a table showing an example of a quantitative sample adjustment criterion stored in a quantitative sample adjustment criterion storage section by the biological sample measurement system shown in FIG. 8;
FIG. 17 is a flowchart showing an example of operation process procedures of a control section of the biological sample measurement system shown in FIG. 8;
FIG. 18 is a flowchart showing an example of a quantitative measurement necessity determination subroutine in the flowchart shown in FIG. 17;
FIG. 19 is a flowchart showing an example of an additional washing necessity determination subroutine in the flowchart shown in FIG. 17; and
FIG. 20 is a flowchart showing an example of a quantitative sample adjustment condition determination subroutine of each measurement item in the flowchart shown in FIG. 17.

Hereinbelow, preferred embodiments of the present invention will be described specifically with reference to the drawings.

### [First Exemplary System]

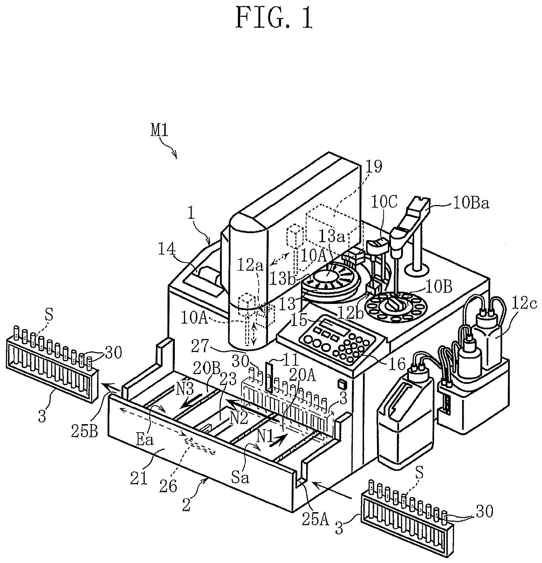

Each of FIGS. 1 to 4 shows an example of a biological sample measurement device. As shown in FIG. 1, a biological sample measurement device M1 of the present example includes a main body portion 1 and a transfer device 2. The biological sample measurement device M1 is installed in, e.g., a laboratory in a hospital.

The biological sample measurement device M1 can be used alone, and can also be used by being connected to other information processing devices and biological sample measurement devices via communication that uses a local area network (LAN) constructed in the hospital or a line such as the Internet.

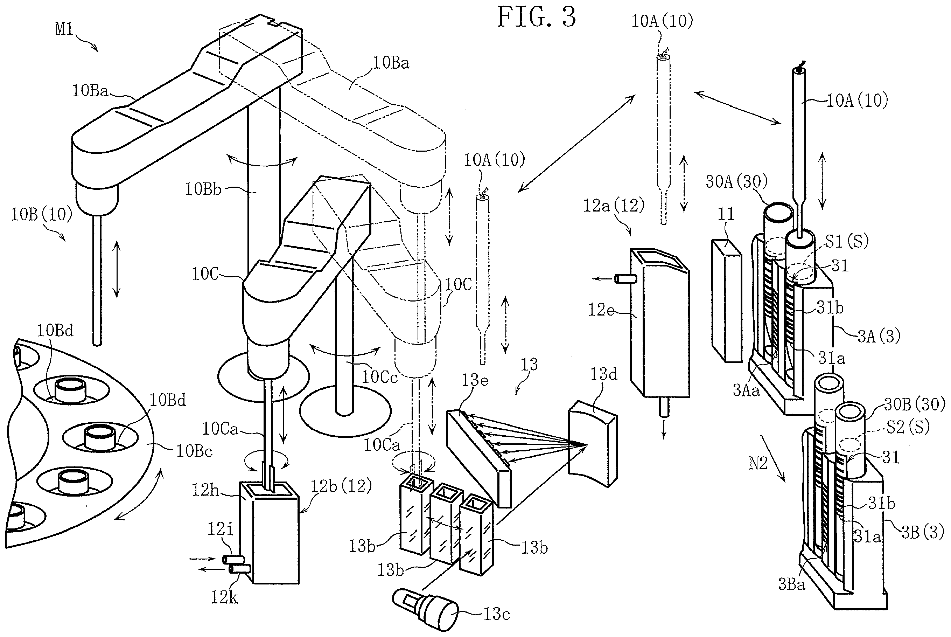

The biological sample measurement device M1 is configured to be capable of performing quantitative measurement of a plurality of types of biological samples S sequentially, and performing the quantitative measurement of a plurality of measurement items of each biological sample S sequentially. Examples of the biological sample S that can be measured include blood serum, blood plasma, and whole blood in addition to urine and feces. With regard to the feces, a liquid obtained by filtering a suspension in which the feces are suspended is used as the sample. As shown in FIGS. 1 and 3, the biological sample S is contained in a sample container 30 on a per type basis. The sample container 30 corresponds to an example of a sample container in the present invention.

As shown in FIG. 3, as the sample container 30, for example, a urine container 30A in which urine S1 is contained and a feces container 30B in which feces S2 are contained are used. These containers 30 are provided with information recording sections 31. The information recording section 31 is configured by using, e.g. , a label 31a on which a bar code 31b is printed. In the information recording section 31, subject identification information for identifying a subject as a provider of the biological sample S is recorded. The subject identification information is, e.g., a subject identification number or a sample number, and is information for associating the biological sample S with the subject.

The sample container 30 is held upright in a rack 3. As the rack 3, for example, a rack 3A for the urine container 30A and a rack 3B for the feces container 30B are prepared. The racks 3A and 3B are also provided with information recording sections 3Aa and 3Ba. Each of the information recording sections 3Aa and 3Ba is configured by using, e.g., a label on which a bar code is printed. It is possible to record information on the type of the biological sample S in the information recording sections 3Aa and 3Ba.

It is possible to include the information on the type of the biological sample S in the information recording section 31. The type of the biological sample S corresponds to an example of a characteristic in the present invention. In this case, it is possible to adopt a configuration in which the urine container 30A and the feces container 30B are held upright in a state in which the urine container 30A and the feces container 30B are held in the same rack 3.

The biological sample measurement device M1 is a device for performing the quantitative measurement on a plurality of types of the biological samples S. The type of the biological sample S is always input at the time of the measurement in order to identify the type of the biological sample S. The information on the type of the biological sample S is input manually by an examiner, or is automatically read from the information recording section 31 of the sample container 30, the information recording section 3Aa of the rack 3A, or the information recording section 3Ba of the rack 3B by an ID reading section 11.

As shown in FIG. 1, the transfer device 2 is provided for transferring the rack 3 that holds the sample container 30 upright using specific paths. The transfer device 2 has two transfer paths 20A and 20B that are separated by a partition stand 23, and a frame portion 21 that surrounds the near side and both sides of the transfer paths 20A and 20B. In the transfer device 2, when the rack 3 is put into a start area Sa from a notched portion 25A provided in the frame portion 21, the rack 3 is transferred in directions indicated by arrows N1 to N3 sequentially, and reaches an end area Ea finally. The rack 3 having reached the end area Ea is pushed out of the transfer device 2 via a notched portion 25B by a pusher 26. Reading of the bar code stuck to the sample container 30 and collection of the biological sample S from the sample container 30 are performed in the process of transferring the rack 3 in the direction indicated by the arrow N2.

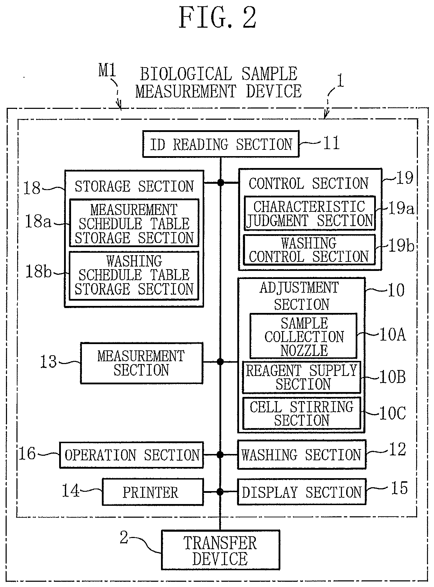

As shown in FIG. 2, the main body portion 1 of the biological sample measurement device M1 includes an adjustment section 10, the ID reading section 11, a washing section 12, a measurement section 13, a printer 14, a display section 15, an operation section 16, a storage section 18, and a control section 19.

The adjustment section 10 is provided for adjusting a reaction liquid for measuring a component contained in the biological sample S in a reaction cell 13b described later. As shown in FIGS. 2 and 3, the adjustment section 10 includes a sample collection nozzle 10A, a reagent supply section 10B, and a cell stirring section 10C.

The sample collection nozzle 10A is provided for sucking and collecting a predetermined quantity of the biological sample S from the sample container 30 having passed the front of the ID reading section 11. As shown in FIG. 3, the collected biological sample S is supplied to the measurement section 13 described later. The sample collection nozzle 10A performs a collection operation of the biological sample S and a dispensing operation to the reaction cell 13b described later with driving by a sample collection nozzle driving section (not shown).

The ID reading section 11 is, e.g., a bar code reader and, as shown in FIGS. 1 and 3, when the sample container 30 is transferred to the front of the ID reading section 11 by the transfer device 2, the ID reading section 11 reads the subject identification information from the information recording section 31 of the sample container 30. By reading the subject identification information, it is possible to confirm the type of the biological sample S and the ordered measurement item of the biological sample S. The reading of the subject identification information by the ID reading section 11 is sequentially performed irrespective of whether or not previously performed measurement is completed.

The reagent supply section 10B is provided for supplying a dilution liquid for diluting the biological sample S and a liquid reagent to the reaction cell 13b. As shown in FIGS. 1 and 3, the reagent supply section 10B includes a reagent dispensing nozzle 10Ba and a reagent table 10Bc. On the reagent table 10Bc, bottles 10Bd filled with the dilution liquid of the biological sample S and the liquid reagent are set. A plurality of types of the liquid reagents are prepared on the reagent table 10Bc, and hence it is possible to perform the measurement of a plurality of the measurement items on one biological sample S. The measurement of each measurement item is performed by using, e.g., a generally known measurement principle. The reagent dispensing nozzle 10Ba moves between the reagent table 10Bc and a reaction turntable 13a by rotating about a support 10Bb, and dispenses the dilution liquid and the liquid reagent to the reaction cell 13b.

The dispensing quantity of the biological sample S to the reaction cell 13b is configured to be changeable. The dispensing quantity of the dilution liquid or the liquid reagent to the reaction cell 13b is also configured to be changeable. It is possible to change a dilution factor of the biological sample S in the reaction liquid by changing at least one of the dispensing quantities of the biological sample S, the dilution liquid, and the liquid reagent. By changing the dilution factor, it is possible to change a measurement range of the measurement item as a target.

The cell stirring section 10C is provided for stirring the reaction liquid including the biological sample S, the dilution liquid, and the liquid reagent dispensed to the reaction cell 13b using a stirring blade 10Ca.

As shown in FIGS. 1 and 3, the measurement section 13 includes the reaction turntable 13a, the reaction cells 13b, a light source lamp 13c, a concave diffraction grating 13d, and an optical receiver 13e. The reaction cells 13b are set at predetermined positions of the reaction turntable 13a.

The liquid reagent is configured to react with a predetermined component contained in the biological sample S, and develop a color of a degree corresponding to the concentration of the component. As shown in FIG. 3, light emitted from the light source lamp 13c passes through the reaction cell 13b as indicated by an arrow, is separated by the concave diffraction grating 13d, and is detected by the optical receiver 13e as absorbance. Based on the absorbance, a quantitative measurement concentration of the predetermined component in the biological sample is calculated. Hereinafter, in the present example, the absorbance and the quantitative measurement concentration are collectively referred to as quantitative measurement values. Note that the absorbance or the quantitative measurement concentration obtained by the measurement by the measurement section 13 is stored in the storage section 18 described later.

As shown in FIGS. 1 and 3, the washing section 12 includes a sample collection nozzle washing section 12a, a stirring blade washing section 12b, and a washing liquid bottle 12c. The sample collection nozzle washing section 12a is provided for washing the sample collection nozzle 10A after the dispensing of the biological sample S. The stirring blade washing section 12b is provided for washing the stirring blade 10Ca after the stirring of the reaction cell 13b. The sample collection nozzle 10A and the stirring blade 10Ca are portions that contact the biological sample S when the biological sample measurement device M1 performs the measurement of the biological sample S, and correspond to examples of a biological sample contact portion in the present invention. Washing is performed in order to avoid occurrence of an influence on measurements that are sequentially performed. Note that the stirring blade washing section 12b is also used for the washing of the reagent dispensing nozzle 10Ba.

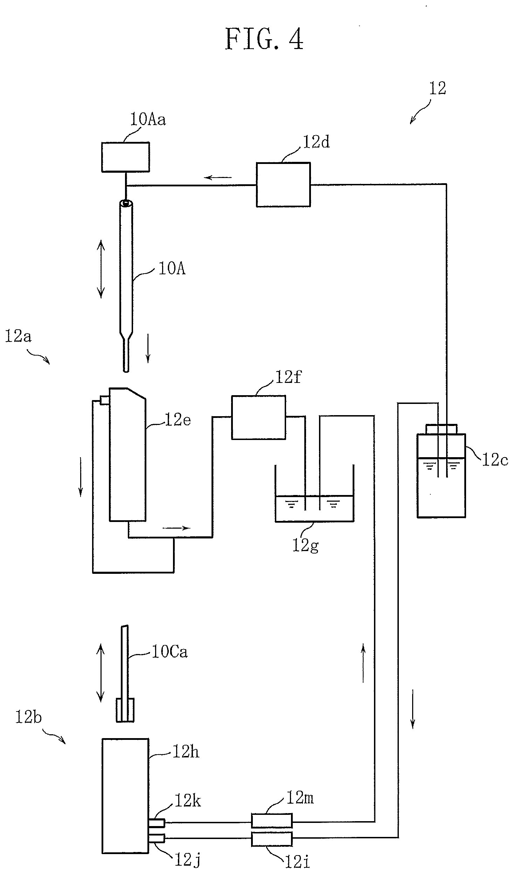

The operation of the washing section 12 is controlled by a washing control section 19b described later. FIG. 4 shows a schematic configuration of the washing section 12. The sample collection nozzle 10A is connected to a spotting pump 10Aa. The spotting pump 10Aa is provided for performing collection dispensing of the biological sample S.

As shown in FIG. 4, in the sample collection nozzle washing section 12a, the sample collection nozzle 10A is connected to the washing liquid bottle 12c. A washing liquid supply driving section 12d is disposed between the sample collection nozzle 10A and the washing liquid bottle 12c. A bottom portion and an upper portion of a sample collection nozzle washing tank 12e are connected to a waste liquid tank 12g via a washing liquid discharge driving section 12f.

After the biological sample S is dispensed to the reaction cell 13b, the sample collection nozzle 10A moves to a position over the sample collection nozzle washing tank 12e, descends, and is accommodated in the sample collection nozzle washing tank 12e. Subsequently, the washing control section 19b switches the connection of the sample collection nozzle 10A from the connection to the spotting pump 10Aa to connection to the washing liquid supply driving section 12d to discharge the washing liquid into the sample collection nozzle washing tank 12e. With this, the inside of the sample collection nozzle 10A is washed. In addition, an outer wall of the sample collection nozzle 10A is also washed with the washing liquid stored in the sample collection nozzle washing tank 12e. On the other hand, after completion of washing of the sample collection nozzle 10A, the washing control section 19b drives the washing liquid discharge driving section 12f to discard the washing liquid into the waste liquid tank 12g.

The washing control section 19b changes a washing liquid quantity and washing time by changing a driving amount of the washing liquid supply driving section 12d. The washing time is changed and washing performance is adjusted by changing a time period in which the washing liquid is stored in the sample collection nozzle washing tank 12e. The washing performance is enhanced by increasing the number of times of the driving of the washing liquid supply driving section 12d.

The stirring blade washing section 12b is provided for washing the stirring blade 10Ca of the cell stirring section 10C. As shown in FIGS. 3 and 4, the stirring blade washing section 12b includes a stirring blade washing tank 12h, a washing liquid supply driving section 12i, a washing liquid supply opening 12j, a washing liquid discharge opening 12k, and a washing liquid discharge driving section 12m. After the reaction liquid in the reaction cell 13b is stirred, the cell stirring section 10C rotates about a support 10Cc to move to a position over the stirring blade washing tank 12h, and descends. With this, the stirring blade 10Ca is accommodated in the stirring blade washing tank 12h. The washing liquid is supplied from the washing liquid supply opening 12j with the driving of the washing liquid supply driving section 12i. The stirring blade 10Ca is washed by rotating in the stirring blade washing tank 12r. When the washing is completed, the washing liquid is discarded from the washing liquid discharge opening 12k into the waste liquid tank 12g with the driving of the washing liquid discharge driving section 12m.

The washing control section 19b changes the washing performance of the stirring blade washing section 12b by changing the washing liquid quantity. The washing time is changed and the washing performance is adjusted by changing a time period in which the washing liquid is stored in the stirring blade washing tank 12h. In addition, the washing performance is adjusted by increasing or decreasing the number of times of the supply of the washing liquid.

The washing control section 19b can also be configured to adjust the washing performance of the stirring blade washing section 12b by changing the type of the washing liquid. In this case, in the washing section 12, a plurality of types of the washing liquids obtained by changing the type of a contained washing agent or changing the concentration of the washing agent are prepared. In the case where the concentration of the washing agent is changed, a configuration may also be adopted in which a dilution liquid for the washing liquid is additionally prepared and the dilution factor is automatically changed on an as needed basis.

In FIGS. 1 and 2, the printer 14 is provided for printing the result of the quantitative measurement of the measurement item performed on the biological sample S on a predetermined paper sheet in association with the subject identification information, and outputting the paper sheet. The display section 15 includes an image display screen such as a liquid crystal display panel, and performs screen display for guiding, e.g., the operation of the operation section 16. Note that the display section 15 may be caused to display the result of the quantitative measurement. The operation section 16 is provided for the examiner to input the subject identification information, the measurement item of each biological sample S, and a calibration curve of each measurement item to the biological sample measurement device M1 in accordance with an operation guide displayed in the display section 15.

The control section 19 is configured by using, e.g., a microcomputer, and includes a characteristic judgment section 19a and the washing control section 19b in particular.

The characteristic judgment section 19a is a section that judges the characteristic of the biological sample S. An example of the characteristic of the biological sample S includes the type of the biological sample S. Specifically, the characteristic judgment section 19a judges, e.g., that the biological sample S is "the urine S1" or "the feces S2".

The judgment of the characteristic by the characteristic judgment section 19a is performed at least before the measurement on the biological sample S to be washed is completed. The completion of the measurement denotes the completion of the measurement of the absorbance of the reaction liquid (the same applies to the following description). Alternatively, the judgment thereof is performed at least before the measurement of the biological sample S to be washed is started. The start of the measurement denotes the start of the reaction between the specific component in the biological sample S and the reagent in the reaction liquid (the same applies to the following description). Alternatively, the judgment thereof is performed at least before the biological sample S to be washed is dispensed to the reaction cell 13b. Alternatively, the judgment thereof is performed at least before the stirring of the reaction liquid in the reaction cell 13b is completed.

The washing control section 19b is a section for determining a washing method by the washing section 12 based on the characteristic judged by the characteristic judgment section 19a. Specifically, the washing control section 19b determines the washing method by the washing section 12 based on the type of the biological sample S. The washing control section 19b changes the washing liquid quantity, the washing time, or the type of the washing liquid based on the determined washing method, and causes the washing section 12 to wash the biological sample contact portion.

The determination of the washing method by the washing control section 19b is performed at least before the measurement of the biological sample S to be washed is completed. Alternatively, the determination thereof is performed at least before the measurement of the biological sample S to be washed is started. Alternatively, the determination thereof is performed at least before the biological sample S to be washed is dispensed to the reaction cell 13b. Alternatively, the determination thereof is performed at least before the stirring of the reaction liquid in the reaction cell 13b is completed.

The control section 19 performs control of an operation process of the main body portion 1 or the transfer device 2, management of the quantitative measurement value acquired by the measurement section 13, and management of output of the quantitative measurement value.

The storage section 18 is configured by using, e.g., a memory, an HDD, or an SSD, and includes a measurement schedule table storage section 18a and a washing schedule table storage section 18b in particular. In addition to them, the storage section 18 stores programs and various data sets for causing the control section 19 to execute operation control of the individual sections of the biological sample measurement device M1 and various data processes. For example, the storage section 18 stores the calibration curve and the quantitative measurement value of each measurement item.

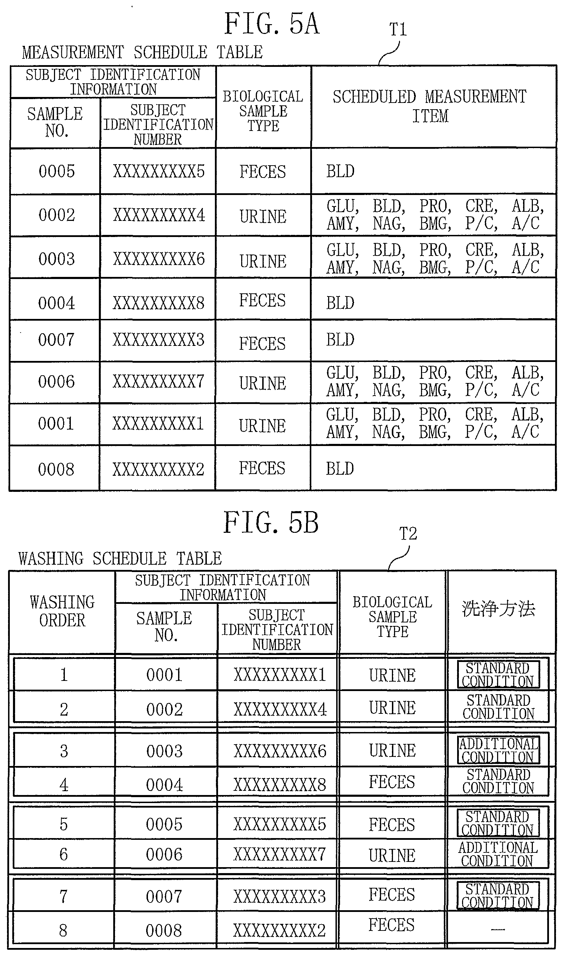

FIG. 5A shows an example of a measurement schedule table stored in the measurement schedule table storage section 18a. As shown in FIG. 5A, a measurement schedule table T1 has, e.g., the section of the subject identification information, the section of the biological sample type, and the section of a scheduled measurement item. These items are input in advance by the examiner by operating the operation section 16 when the measurement is started. The measurement schedule table T1 includes pair information in which the subject identification information is paired with the type of the biological sample S corresponding to the subject identification information. The measurement schedule table T1 includes the subject identification number and a sample number as the subject identification information. Note that the biological sample measurement schedule table T1 indicates a state before a measurement order is determined. The measurement order is determined in accordance with, e.g., the order in which the subject identification information recorded in the sample container 30 is read by the ID reading section 11. In addition, as described above, the information on the type of the biological sample S may be information manually input by the examiner, and may also be information that is automatically read from the information recording section 31 of the sample container 30 or the information recording sections 3Aa and 3Ba of the rack 3 and stored in the measurement schedule table storage section 18a.

In the measurement schedule table T1, in the case where the biological sample S is the urine S1, as the measurement items, glucose (GLU), occult blood (BLD), protein (PRO), creatinine (CRE), albumin (ALB), amylase (AMY), β-D-N acetylglucosaminidase (NAG), β2-microglobulin (BMG), urinary protein/creatinine ratio (P/C), and albumin/creatinine ratio (A/C) are measured. On the other hand, in the case where the biological sample S is the feces S2, occult blood (BLD) is measured as the measurement item.

FIG. 5B shows an example of a washing schedule table stored in the washing schedule table storage section 18b. As shown in FIG. 5B, a washing schedule table T2 has, e.g., the section of a washing order, the section of the subject identification information, the section of the biological sample type, and the section of the washing method. Every time the ID reading section 11 reads the subject identification information, the characteristic judgment section 19a identifies the corresponding type of the biological sample S by referring to the measurement schedule table T1 based on the subject identification information, and determines the type thereof as the characteristic. The type of the biological sample S corresponds to an example of the characteristic of the biological sample in the present invention. The characteristic judgment section 19a writes the washing order and the type of the biological sample S into the washing schedule table T2. The washing control section 19b refers to the washing schedule table T2 to determine the washing method of each biological sample S, and writes the washing method into the section of the washing method in the washing schedule table T2.

A urine occult blood test is a test for determining the presence or absence of hemorrhage in a kidney urinary tract system with a hemoglobin concentration in the urine S1 used as an index. The urine occult blood test is used as indexes of an inflammation, tumor, and stone in a kidney, ureter, bladder, urethra, and prostate. In contrast to this, a fecal occult blood test is a test for determining the presence or absence of hemorrhage in the digestive tract with the hemoglobin concentration in the feces S2 used as an index. The fecal occult blood test is performed mainly for the purpose of early detection of colorectal cancer.

In the present example, in the measurement of the urine occult blood and the fecal occult blood, the common adjustment section 10, the common washing section 12, the common measurement section 13, and the common reagent are used. However, it is well known that the concentration of the occult blood in the urine S1 is 1000 or more times higher than the concentration thereof in the feces S2. Accordingly, in the washing with a usual condition, there are cases where a blood component of the urine S1 remains in the biological sample contact portion such as the flow path of the biological sample S. In particular, in the case where the fecal occult blood is measured subsequently to the measurement of the urine occult blood, it is feared that false determination (false-positive) occurs. In this case, it is necessary to perform the washing with an additional condition in which the washing performance is enhanced.

For example, in the washing schedule table T2 in FIG. 5B, when attention is paid to the biological sample types of washing orders 1 and 2, the urine S1 is measured subsequently to the measurement of the urine S1. In this case, the urine S1 of a sample number 0001 is washed with a standard condition. This is because, in such a case, clinically accurate washing is not required.

Next, when attention is paid to the biological sample types of washing orders 3 and 4, the feces S2 are measured subsequently to the measurement of the urine S1. In this case, the urine S1 of a sample number 0003 is washed with the additional condition. As described above, this is because the concentration of the fecal occult blood is lower than the concentration of the urine occult blood and the clinically accurate measurement is required.

Next, when attention is paid to the biological sample types of washing orders 5 and 6, the urine S1 is measured subsequently to the measurement of the feces S2. In this case, the feces S2 of a sample number 0005 is washed with the standard condition. The concentration of the fecal occult blood is lower than the concentration of the urine occult blood, and hence standard washing is adequate.

Further, when attention is paid to the biological sample types of washing orders 7 and 8, the feces S2 are measured subsequently to the measurement of the feces S2. In this case, the feces S2 of a sample number 0007 is washed with the standard condition. Similarly to the above case, the concentration of the fecal occult blood is lower than the concentration of the urine occult blood, and hence the standard washing is adequate.

It goes without saying that the washing control section 19b can determine the washing condition based on the type of the biological sample S without considering the relationship with the type of the biological sample S that is measured next.

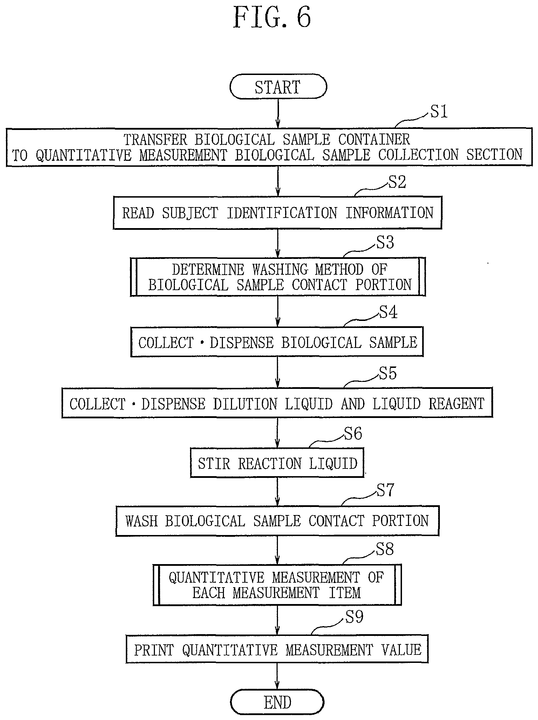

Next, a description will be given of an example of operation process procedures of the biological sample measurement device M1 by the control section 19 with reference to flowcharts shown in FIGS. 6 and 7 and FIGS. 1 to 5.

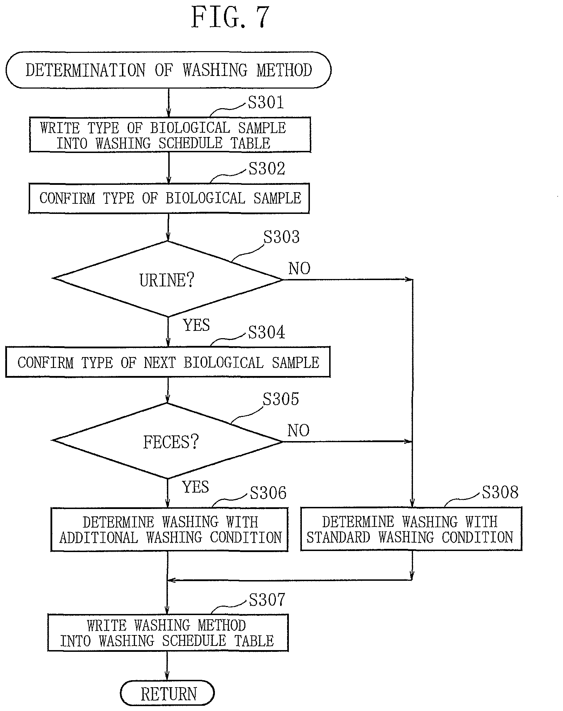

As shown in the flowchart in FIG. 6, when the rack 3 that holds the sample container 30 in which the biological sample S is contained is put into the transfer device 2, the rack 3 is transferred toward a biological sample collection section 27 that is positioned midway in a transfer path indicated by the arrow N2 (S1). While the rack 3 is transferred toward the biological sample collection section 27, the bar code 31b of the sample container 30 is read by the ID reading section 11 (S2). Next, the determination of the washing method of the biological sample contact portion is performed (S3). This operation process is performed in accordance with, e.g., a subroutine shown in FIG. 7.

FIG. 7 shows an example of the way of determining the washing method in the case where the urine S1 and the feces S2 are present as the biological sample S. Every time the ID reading section 11 reads the subject identification information, the characteristic judgment section 19a identifies the corresponding type of the biological sample S by referring to the measurement schedule table T1 based on the subject identification information. When the type of the biological sample S is identified, the characteristic judgment section 19a writes the washing order and the type of the biological sample S into the washing schedule table T2 (S301).

Next, the washing control section 19b refers to the washing schedule table T2 to confirm the type of the biological sample S (S302). In the case where the type of the biological sample S is the urine S1, the type of the biological sample that is measured next is confirmed (S303: YES, S304). In the case where the type of the next biological sample S is the feces S2, the washing control section 19b determines the execution of the washing with the additional condition, and writes the determination into the washing schedule table T2 (S305: YES, S306, S307).

In the case where the washing control section 19b refers to the washing schedule table T2 to determine that the type of the biological sample S is not the urine S1 (i.e., the feces S2), the washing control section 19b determines the execution of the washing with the standard condition, and writes the determination into the washing schedule table T2 (S303: NO, S308, S307) . In addition, in the case where the washing control section 19b determines that the type of the next biological sample S is not the feces S2 (i.e., the urine S1), the washing control section 19b determines the execution of the washing with the standard condition, and writes the determination into the washing schedule table T2 (S305: NO, S308, S307). When the above processes are completed, the procedures return to the flowchart shown in FIG. 6.

Next, the sample collection nozzle 10A moves to the measurement section 13 and dispenses the biological sample S to the reaction cell 13b after the sample collection nozzle 10A collects the biological sample S in the biological sample collection section 27 (S4). Next, the reagent dispensing nozzle 10Ba dispenses the dilution liquid and the liquid reagent to the reaction cell 13b (S5). Next, the cell stirring section 10C stirs a liquid mixture in the reaction cell (S6).

Next, the washing control section 19b refers to the washing schedule table T2 to cause the washing section 12 to wash the sample collection nozzle 10A and the stirring blade 10Ca that have contacted the biological sample S (S7) . The washing section 12 can start the washing before the measurement of the biological sample S is completed. For example, the washing of the sample collection nozzle 10A is started at the point of time when the washing method of the biological sample S is determined and the dispensing to the reaction cell 13b is completed. In addition, the washing of the stirring blade 10Ca is started at the point of time when the washing method of the biological sample S is determined and the stirring of the reaction liquid of the reaction cell 13b is completed.

Next, as described above, the measurement section 13 performs the quantitative measurement of the measurement item specified for each biological sample S (S8).

When the quantitative measurement of each measurement item of the biological sample S is completed, the printer 14 prints the quantitative measurement value in association with the subject identification information (S9).

According to the present example, the type of the biological sample S is adopted as the characteristic when the washing control section 19b determines the washing method. The type of the biological sample S is information that can be acquired before the measurement of the biological sample S is performed. Accordingly, the washing control section 19b can early determine whether or not additional washing of the biological sample contact portion is necessary. In addition, the washing control section 19b can select the proper washing condition in accordance with the type of the biological sample S instead of using the uniform washing condition. With this, it is possible to achieve acceleration of the measurement while maintaining accuracy in the measurement by the measurement section 13.

According to the present example, the type of the biological sample S is adopted as the characteristic of the biological sample S. In the biological sample measurement device M1, the type of the biological sample S is always input at the time of the measurement. The characteristic judgment section 19a uses the information when the characteristic judgment section 19a judges the characteristic of the biological sample S. Accordingly, it is not necessary to add a new detection device or the like in order to obtain the characteristic. With this, it is possible to prevent an increase in the manufacturing cost of the biological sample measurement device M1.

### [First Embodiment]

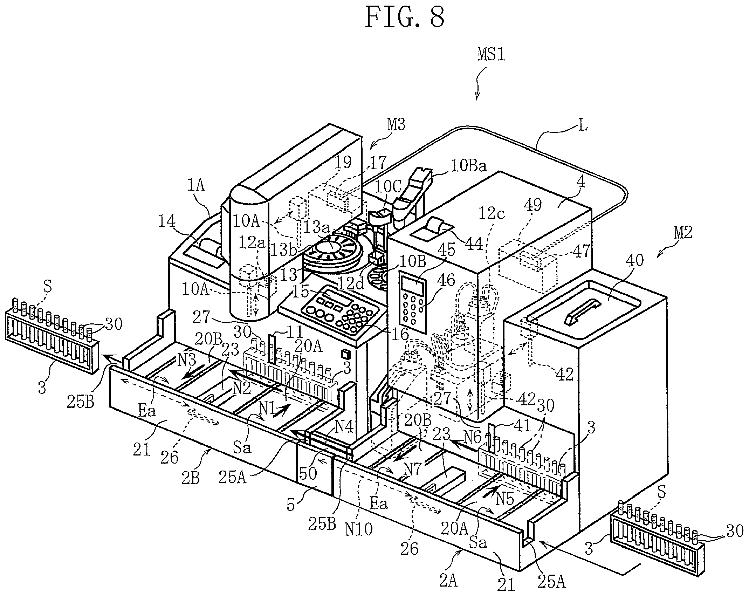

Each of FIGS. 8 to 10 shows an example of a biological sample measurement system according to the present invention. In the drawings, elements identical or similar to those in the first example are designated by the same reference numerals as those in the first example. The detailed description of the elements designated by the same reference numerals will be omitted.

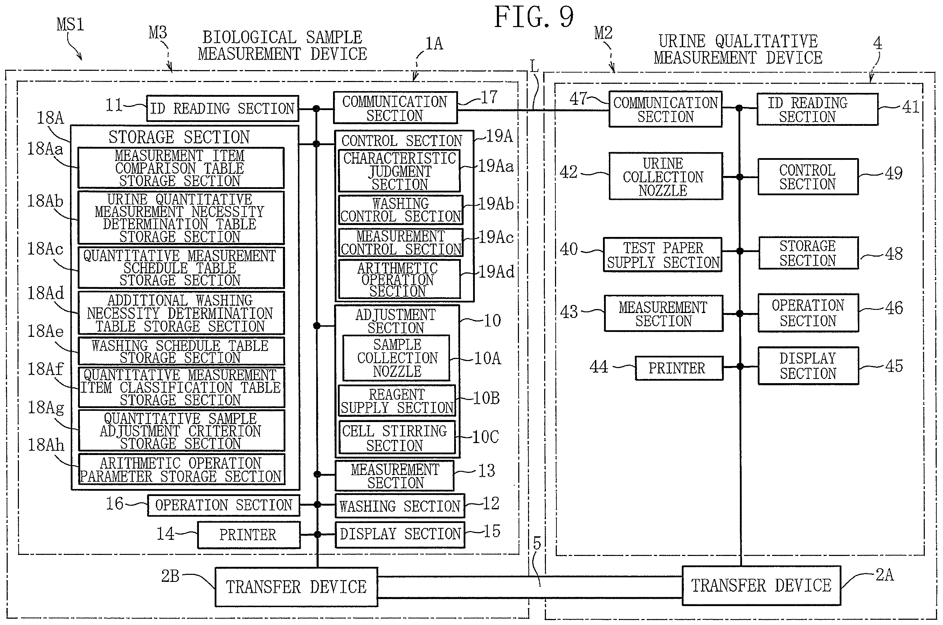

A measurement system MS1 is installed in, e.g., a laboratory of a hospital, and is used to perform the urine quantitative measurement successively after urine qualitative measurement is performed. As shown in FIGS. 8 and 9, the measurement system MS1 includes a urine qualitative measurement device M2 and a biological sample measurement device M3. The urine qualitative measurement device M2 is provided for performing the urine qualitative measurement on urine by using a urine test paper and acquiring a urine qualitative measurement result of a predetermined measurement item. In contrast to this, the biological sample measurement device M3 is configured to be capable of measuring not only the urine but also a plurality of types of the biological samples S other than the urine, and measure a plurality of measurement items of each biological sample sequentially. Examples of the biological sample that can be measured include feces, blood serum, blood plasma, and whole blood in addition to urine. With regard to the feces, a liquid obtained by filtering a suspension in which the feces are suspended is used as the sample.

In the following description, a description will be given of the case where the urine S1 and the feces S2 are measured as examples of the biological sample S. (The urine S1 and the feces S2 are the same as those described in the first example.)

The urine qualitative measurement device M2 and the biological sample measurement device M3 are directly connected to each other via a communication line L. Note that the urine qualitative measurement device M2 and the biological sample measurement device M3 may be capable of data communication mutually between them appropriately, and they can be configured to be connected to each other via communication that uses a local area network (LAN) constructed in, e.g., the hospital or a line such as the Internet.

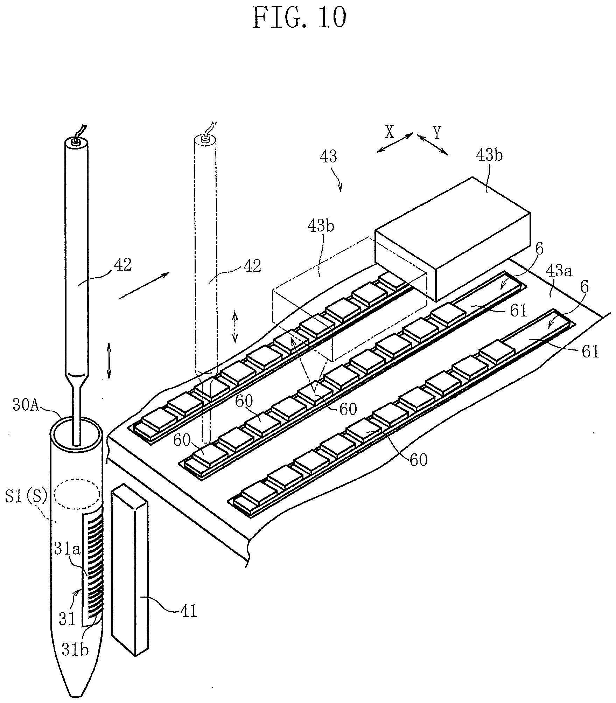

As shown in FIG. 10, the urine S1 is contained in the urine container 30A. As described in the first example, the urine container 30A is held upright in the rack 3. Note that, in the rack 3, it is possible to hold a container (e.g., the feces container 30B in which the feces S2 are contained) in which the biological sample S of the type different from the urine S1 is contained upright together with the urine container 30A. As shown in FIG. 10, the urine container 30A is provided with the information recording section 31 in which the subject identification information is recorded, as described above. The urine container 30A corresponds to an example of the sample container in the present invention. Note that the urine container 30A may also be held upright in the dedicated rack 3A described in the first example. The feces container 30B can also be held upright in the dedicated rack 3B described in the first example.

As shown in FIG. 8, the urine qualitative measurement device M2 is provided for performing a qualitative measurement process of the urine S1 contained in the urine container 30A, and corresponds to an example of external equipment in the present invention. The urine qualitative measurement device M2 has a main body portion 4 and a transfer device 2A.

Note that the external equipment is not limited to the urine qualitative measurement device M2. The external equipment may also be an information processing device such as a personal computer that is directly connected to the biological sample measurement device M3. In addition, the external equipment may also be an information processing device such as a server that is connected to a local area network (LAN) constructed in the hospital or the Internet.

The transfer device 2A is a device similar to the transfer device 2 described above, and transfers the rack 3 in directions indicated by arrows N5 to N7 sequentially. Reading of the bar code of the urine container 30A and the collection of the urine S1 are performed in the process of transferring the rack 3 in the direction indicated by the arrow N6. The transfer device 2A is connected to a transfer device 2B of the biological sample measurement device M3 described later via a connection member 5. The pusher 26 reciprocates along a front surface of the frame portion 21 in a direction indicated by an arrow N10 to move the rack 3. The rack 3 moves to the transfer device 2B through a transfer path 50 of the connection member 5 as indicated by the arrow N4 by being pushed by the pusher 26. Note that the rack 3 that holds the feces container 30B in which the feces S2 are contained upright may be put in the transfer device 2A. In addition, the rack 3B dedicated to the feces container 30B may also be transferred. In these cases, by identifying the type of the biological sample S, collection of the feces S2 from the feces container 30B is prevented in the urine qualitative measurement device M2.

As shown in FIG. 9, the main body portion 4 of the urine qualitative measurement device M2 includes a test paper supply section 40, an ID reading section 41, a urine collection nozzle 42, a measurement section 43, a printer 44, a display section 45, an operation section 46, a communication section 47, a storage section 48, and a control section 49.

As shown in FIG. 10, the urine qualitative measurement by the urine qualitative measurement device M2 is performed by using a multi-item test piece 6 as the urine test paper. In the multi-item test piece 6, a plurality of reagent pads 60 are disposed on a plastic stick 61. The multi-item test piece 6 is preserved in a bottle (not shown) . The multi-item test piece 6 is taken out of the bottle when it is used, and is put into the test paper supply section 40 shown in FIG. 8. When a measurement operation is started, as shown in FIG. 10, the multi-item test piece 6 is automatically placed on a placement stand 43a constituting the measurement section 43 described later.

According to the multi-item test piece 6, with different reagents contained in the individual reagent pads 60, it is possible to perform the urine qualitative measurement of a plurality of measurement items simultaneously. Examples of the measurement item that can be measured by using the multi-item test piece 6 include glucose (GLU), protein (PRO), urobilinogen (URO), bilirubin (BIL), creatinine (CRE), pH, occult blood (BLD), ketone body (KET), nitrite (NIT), leukocyte (LEU), albumin (ALB), urinary protein/creatinine ratio (P/C), and albumin/creatinine ratio (A/C) . The measurement of each measurement item is performed by using, e.g., a generally known measurement principle. With the use of the multi-item test piece 6, it is possible to acquire qualitative values or semi-quantitative values of these measurement items. The qualitative value and the semi-quantitative value correspond to examples of a urine qualitative measurement result in the present invention.

The ID reading section 41 is, e.g., a bar code reader and, as shown in FIGS. 8 and 10, the ID reading section 41 is provided for reading the subject identification information from the information recording section 31 of the urine container 30A transferred by the transfer device 2A. The subject identification information is sent to the biological sample measurement device M3 from the communication section 47 together with information on the type of the biological sample S. The information on the type of the biological sample S may be information manually input by the examiner, and may also be information automatically read by the ID reading section 41 from the information recording section 31 of the sample container 30, the information recording section 3Aa of the rack 3A, or the information recording section 3Ba of the rack 3B. The control section 49 confirms the ordered measurement item of the urine S1 by reading the subject identification information. The urine collection nozzle 42 is provided for sucking and collecting a predetermined quantity of the urine S1 from the urine container 30A transferred to the urine collection section 27. The collected urine S1 is supplied to the measurement section 43 described later.

As shown in FIG. 10, the measurement section 43 includes the placement stand 43a and an optical measuring instrument 43b. As described above, the multi-item test piece 6 is placed on the placement stand 43a. The predetermined quantity of the urine S1 collected by the urine collection nozzle 42 is dispensed onto each of a plurality of the reagent pads 60 of the multi-item test piece 6. Each of the plurality of the reagent pads 60 is configured to react with a predetermined component in the urine S1 and develop a color of a degree corresponding to the concentration of the component. The optical measuring instrument 43b is configured to be movable in directions X and Y and be capable of measuring the degree of the color development of each reagent pad 60 to which the urine S1 has been dispensed as optical reflectance or the like. The concentration of the predetermined component in the urine S1 is calculated based on measurement data acquired by the optical measuring instrument 43b. The measurement data such as the optical reflectance also corresponds to an example of the urine qualitative measurement result in the present invention.

The control section 49 is configured by using, e.g., a microcomputer. The control section 49 is provided for controlling operation processes of the individual sections of the urine qualitative measurement device M2. The storage section 48 is configured by using, e.g., a memory, an HDD, or an SSD, and stores programs and various data sets for causing the control section 49 to execute operation control of the individual sections of the urine qualitative measurement device M2 and various data processes. In addition, the storage section 48 temporarily stores, e.g., the urine qualitative measurement result acquired by the measurement section 43.

The printer 44, the display section 45, and the operation section 46 are elements having functions identical or similar to those of the printer 14, the display section 15, and the operation section 16 in the biological sample measurement device M1 in the first example described above. Therefore, herein, the description thereof will be omitted.

The biological sample measurement device M3 is provided for performing the quantitative measurement process of the urine S1 or the feces S2, and, as shown in FIGS. 8 and 9, the biological sample measurement device M3 has a main body portion 1A and the transfer device 2B. In addition, similarly to the biological sample measurement device M1 described in the first example, the biological sample measurement device M3 is configured to be capable of performing the quantitative measurement of, e.g., blood serum, blood plasma, and whole blood in addition to the urine S1 and the feces S2.

The transfer device 2B is also a device similar to the transfer device 2 described above, and transfers the rack 3 pushed out of the transfer device 2A in directions indicated by arrows N1 to N3 sequentially. The rack 3 reaches an end area Ea of the transfer path 20B finally. Similarly to the transfer device 2, the reading of the bar code of the urine container 30A and the collection of the urine S1 are performed in the process of transferring the rack 3 in the direction indicated by the arrow N2 . The rack 3 is pushed out of the transfer device 2B by the pusher 26 finally. In the transfer device 2B, the feces container 30B is also transferred similarly. In addition, the transfer device 2B is configured such that the biological sample S that is measured only in the biological sample measurement device M3 can be put into the transfer device 2B.

As shown in FIG. 9, the main body portion 1A of the biological sample measurement device M3 includes the adjustment section 10, the ID reading section 11, the measurement section 13, the printer 14, the display section 15, the operation section 16, a communication section 17, a storage section 18A, and a control section 19A. Among them, the adjustment section 10, the ID reading section 11, the printer 14, the display section 15, and the operation section 16 have functions identical or similar to those of the corresponding components in the biological sample measurement device M1 of the first example described above. Therefore, herein, the detailed description thereof will be omitted.

Note that, similarly to the case of the biological sample measurement device M1, the dispensing quantity of the biological sample S to the reaction cell 13b by the sample collection nozzle 10A in the adjustment section 10 is configured to be changeable. In addition, the dispensing quantity of the dilution liquid or the liquid reagent to the reaction cell 13b is also configured to be changeable. Therefore, it is possible to change the dilution factor of the biological sample S in the reaction liquid by changing at least one of the dispensing quantities of the biological sample S, the dilution liquid, and the liquid reagent. In the biological sample measurement device M3 as well, it is possible to change the measurement range of the measurement item as a target by changing the dilution factor.

The control section 19A is configured by using, e.g., a microcomputer and, as shown in FIG. 9, in particular, the biological sample measurement device M3 is different from the biological sample measurement device M1 of the first example in that the control section 19A includes a characteristic judgment section 19Aa, a washing control section 19Ab, a measurement control section 19Ac, and an arithmetic operation section 19Ad.

The characteristic judgment section 19Aa is provided for, e.g., judging the characteristic of the biological sample S. An example of the characteristic includes the type of the biological sample S. Specifically, the characteristic judgment section 19Aa determines the urine S1 or the feces S2 as the characteristic of the biological sample S. In addition to this, an example of the additional characteristic includes the urine qualitative measurement result acquired in the urine qualitative measurement device M2. Further, specifically, an example thereof includes a level of the urine qualitative measurement result. The level of the urine qualitative measurement result is represented by the qualitative value or the semi-quantitative value. The level of the urine qualitative measurement result also corresponds to an example of the characteristic of the biological sample in the present invention.

The judgment of the characteristic by the characteristic judgment section 19Aa is performed at least before the measurement of the urine S1 or the feces S2 to be washed is completed. Alternatively, the judgment thereof is performed at least before the measurement of the urine S1 or the feces S2 to be washed is started. Alternatively, the judgment thereof is performed at least before the urine S1 or the feces S2 to be washed is dispensed to the reaction cell 13b. Alternatively, the judgment thereof is performed at least before the stirring of the reaction liquid including the urine S1 or the feces S2 to be washed, the dilution liquid, and the liquid reagent is completed.

The washing control section 19Ab is a section for determining the washing method by the washing section 12 based on the characteristic judged by the characteristic judgment section 19Aa. Specifically, the washing control section 19Ab is a section for determining the washing method by the washing section 12 based on the type of the biological sample S. In addition, the washing control section 19Ab is a section for determining the washing method by the washing section 12 based on the level of the urine qualitative measurement result obtained from the urine S1. In the case where the level of the urine qualitative measurement result is not less than a predetermined threshold value, the washing control section 19Ab causes the washing section 12 to wash the biological sample contact portion with a condition in which the washing liquid quantity or the washing time is changed.

The determination of the washing method by the washing control section 19Ab is performed at least before the measurement of the urine S1 or the feces S2 to be washed is completed. Alternatively, the determination thereof is performed at least before the measurement of the urine S1 or the feces S2 to be washed is started. Alternatively, the determination thereof is performed at least before the urine S1 or the feces S2 to be washed are dispensed to the reaction cell 13b. Alternatively, the determination thereof is performed at least before the stirring of the reaction liquid including the urine S1 or the feces S2 to be washed, the dilution liquid, and the liquid reagent is completed.

The measurement control section 19Ac is provided for controlling the measurement in the measurement section 13. For example, in the case where the level of the urine qualitative measurement result acquired by the urine qualitative measurement device M2 is not less than the predetermined threshold value, the measurement control section 19Ac causes the measurement section to perform the quantitative measurement.

The measurement section 13 is configured to use a common measurement reagent irrespective of the urine qualitative measurement result acquired by the urine qualitative measurement device M2 or irrespective of the type of the biological sample S. The measurement control section 19Ac controls the measurement method of the quantitative measurement in the measurement section 13 based on the level of the urine qualitative measurement result acquired by the urine qualitative measurement device M2. For example, when attention is paid to the occult blood (BLD), the common liquid reagent is used for the urine S1 and the feces S2. In addition, with regard to BLD of urine S1, the measurement method of the quantitative measurement is controlled based on the level of the urine qualitative measurement result.

The arithmetic operation section 19Ad corrects the absorbance or the quantitative measurement concentration acquired by the biological sample measurement device M3 based on an arithmetic operation parameter stored in an arithmetic operation parameter storage section 18Ah described later. In the present embodiment, the absorbance and the quantitative measurement concentration are collectively referred to as the quantitative measurement values.

In addition to this, the control section 19A performs control of an operation process of the main body portion 1A or the transfer device 2B, management of the quantitative measurement value acquired by the measurement section 13, and management of output of the quantitative measurement value.

The storage section 18A is configured by using, e.g., a memory, an HDD, or an SSD and, in particular, the storage section 18A includes a measurement item comparison table storage section 18Aa, a urine quantitative measurement necessity determination table storage section 18Ab, a quantitative measurement schedule table storage section 18Ac, an additional washing necessity determination table storage section 18Ad, a washing schedule table storage section 18Ae, a quantitative measurement item classification table storage section 18Af, a quantitative sample adjustment criterion storage section 18Ag, and the arithmetic operation parameter storage section 18Ah. In addition to these, the storage section 18A stores programs and various data sets for causing the control section 19A to execute the operation control of the individual sections of the biological sample measurement device M3 and various data processes. For example, the storage section 18A stores the calibration curve, the absorbance, and the quantitative measurement concentration of each measurement item.

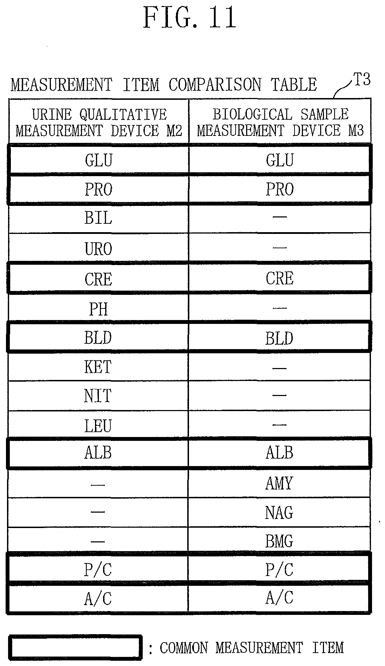

FIG. 11 shows an example of a measurement item comparison table stored in the measurement item comparison table storage section 18Aa. A measurement item comparison table T3 has the section of the measurement item of the urine qualitative measurement device M2 and the section of the measurement item of the biological sample measurement device M3. As described above, examples of the measurement item of the urine qualitative measurement device M2 include GLU, PRO, BIL, URO, CRE, PH, BLD, KET, NIT, LEU, ALB, P/C, and A/C. On the other hand, examples of the measurement item of the biological sample measurement device M3 include GLU, PRO, CRE, BLD, ALB, AMY, NAG, BMG, P/C, and A/C. In this case, as is apparent from FIG. 11, the measurement items common to the urine qualitative measurement device M2 and the biological sample measurement device M3 are GLU, PRO, CRE, BLD, ALB, P/C, and A/C. In the case where the item of the urine qualitative measurement is one of these seven items, the measurement control section 19Ac recognizes that the quantitative measurement necessity determination of the measurement item can be performed based on the urine qualitative measurement result acquired in the urine qualitative measurement device M2. Note that, in these seven items, the measurement principle of the urine qualitative measurement and the measurement principle of the urine quantitative measurement do not need to be identical to each other.

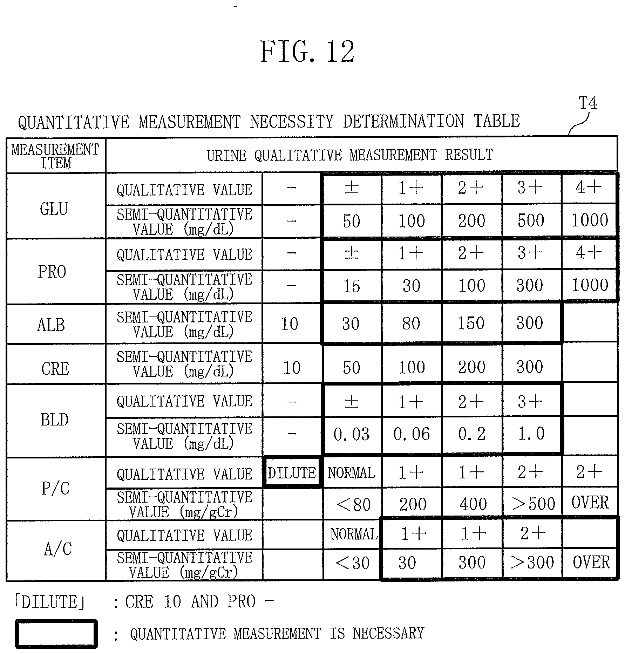

FIG. 12 shows an example of a urine quantitative measurement necessity determination table stored in the urine quantitative measurement necessity determination table storage section 18Ab. In a urine quantitative measurement necessity determination table T4 shown in FIG. 12, when attention is paid to, e.g., BLD, the measurement control section 19Ac automatically determines that the urine quantitative measurement is necessary on the condition that the qualitative value is not less than ± (the semi-quantitative value 0.03) . In the case of GLU, the measurement control section 19Ac determines that the quantitative measurement is necessary, e.g., on the condition that the qualitative value is not less than ± (the semi-quantitative value 50). In the case of PRO, the measurement control section 19Ac determines that the quantitative measurement is necessary, e.g., on the condition that the qualitative value is not less than ± (the semi-quantitative value 15) . In the case where the measurement item is P/C, the measurement control section 19Ac determines that the quantitative measurement is necessary on the condition that the qualitative value is "DILUTE". In this case, the measurement control section 19Ac causes the biological sample measurement device M3 to perform the quantitative measurement of PRO and CRE. Otherwise, the measurement control section 19Ac automatically determines that the quantitative measurement is not necessary. Note that the necessity of the quantitative measurement can also be determined by the examiner independently.

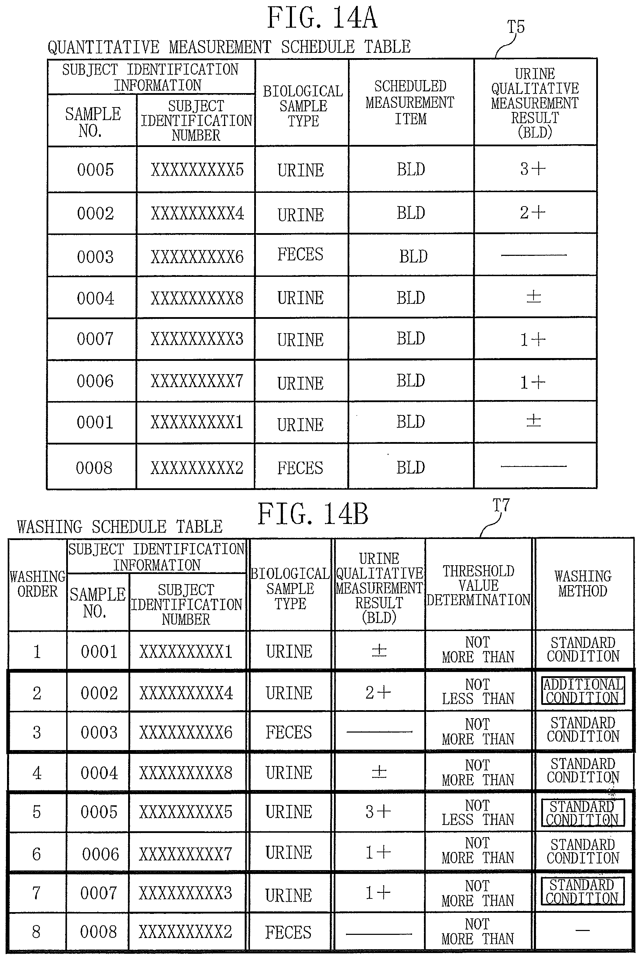

FIG. 14A shows an example of a quantitative measurement schedule table stored in the quantitative measurement schedule table storage section 18Ac. As shown in FIG. 14A, a quantitative measurement schedule table T5 has the section of the subject identification information, the section of the biological sample type, and the section of a scheduled measurement item. These items are input by the examiner by operating the operation section 16 in advance. Examples of the subject identification information include the subject identification number and the sample number, and is information for associating the biological sample S with the subject. The quantitative measurement schedule table T5 has the section of the urine qualitative measurement result. In this section, the urine qualitative measurement result sent from the urine qualitative measurement device M2 is automatically recorded after the completion of the urine qualitative measurement. The quantitative measurement schedule table T5 includes pair information in which the subject identification information is paired with the urine qualitative measurement result corresponding to the subject identification information. A specific example of the urine qualitative measurement result includes the qualitative value or the semi-quantitative value. The urine qualitative measurement result corresponds to another example of the characteristic in the present invention. The quantitative measurement schedule table T5 describes only the result of BLD as an example, but the results of other items are also recorded similarly. In the case where the biological sample S is, e.g., the feces S2, the urine qualitative measurement is not performed, and hence the result is not recorded.

Note that the quantitative measurement schedule table T5 shows a state before the measurement order in the biological sample measurement device M3 is determined. The measurement order is determined in accordance with, e.g., the order in which the subject identification information recorded in the urine container 30A is read by the ID reading section 11.

Information on the type of the biological sample S is not limited to information directly input by the examiner, and may also be information received from the urine qualitative measurement device M2. In addition, the information thereon may also be information that is automatically read by the ID reading section 11 from the information recording section 31 of the sample container 30, the information recording section 3Aa of the rack 3A, or the information recording section 3Ba of the rack 3B.

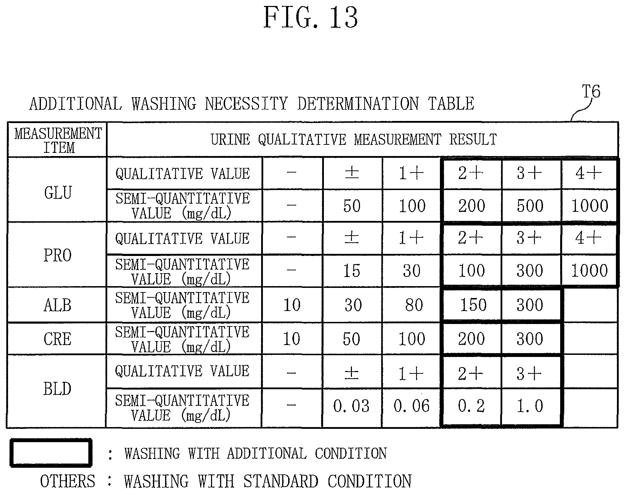

FIG. 13 shows an example of an additional washing necessity determination table stored in the additional washing necessity determination table storage section 18Ad. An additional washing necessity determination table T6 shown in FIG. 13 shows a threshold value. The threshold value corresponds to an example of a first threshold value in the present invention. For example, when attention is paid to BLD, the threshold value is present between the qualitative value 1+ and the qualitative value 2+. That is, the washing control section 19Ab automatically determines that the washing is performed with the additional condition on the condition that the level of the urine qualitative measurement result is not less than the qualitative value 2+ (the semi-quantitative value 0.2) . In contrast to this, in the case where the level of the urine qualitative measurement result is not more than the qualitative value 1+ (the semi-quantitative value 0.06), the washing control section 19Ab automatically determines that the washing is performed with the standard condition. The washing control section 19Ab performs the determination similarly in the case of GLU, PRO, ALB, or CRE.

FIG. 14B shows an example of a washing schedule table stored in the washing schedule table storage section 18Ae. As shown in FIG. 14B, a washing schedule table T7 has the section of the washing order, the section of the subject identification information, the section of the biological sample type, the section of the urine qualitative measurement result, the section of a threshold value determination, and the section of the washing method. Every time the ID reading section 11 reads the subject identification information, the characteristic judgment section 19Aa refers to the quantitative measurement schedule table T5 shown in FIG. 14A based on the subject identification information. The characteristic judgment section 19Aa identifies the corresponding biological sample type and the corresponding urine qualitative measurement result from the quantitative measurement schedule table T5, and writes the washing order, the biological sample type, and the urine qualitative measurement result into the washing schedule table T7. Further, the characteristic judgment section 19Aa refers to the additional washing necessity determination table T6, determines whether the level of the urine qualitative measurement result is not less than a predetermined threshold value or not more than the predetermined threshold value with the level of the urine qualitative measurement result used as the characteristic, and writes the result of the determination into the section of the threshold value determination. The item indicating that the level of the urine qualitative measurement result is not less than the predetermined threshold value or not more than the predetermined threshold value is also considered to correspond to an example of the characteristic of the biological sample in the present invention. The washing control section 19Ab refers to the washing schedule table T7 to determine the washing method for each biological sample, and additionally writes the washing method into the washing schedule table T7.

For example, in the washing schedule table T7 in FIG. 14B, when attention is paid to the biological sample types of washing orders 2 and 3, the feces S2 of a sample number 0003 is measured subsequently to the measurement of the urine S1 of a sample number 0002. In addition, the urine qualitative measurement result of the urine S1 of the sample number 0002 is not less than the threshold value. In this case, the urine S1 of the sample number 0002 is washed with the additional condition. This is because the concentration of the fecal occult blood is lower than the concentration of the urine occult blood, and clinically accurate measurement is required.

Next, when attention is paid to the biological sample types of washing orders 7 and 8, the feces S2 of a sample number 0008 are measured subsequently to the measurement of the urine S1 of a sample number 0007. In addition, the urine qualitative measurement result of the urine S1 of the sample number 0007 is not more than the threshold value. In this case, the urine S1 of the sample number 0007 is washed with the standard condition. From the urine qualitative measurement result, it is predicted that the concentration of the urine occult blood is low, and an influence on the quantitative measurement of the feces S2 of the sample number 0008 is small, and hence standard washing is considered to be adequate.

Next, when attention is paid to the biological sample types of washing orders 5 and 6, the urine S1 of a sample number 0006 is measured subsequently to the measurement of the urine S1 of a sample number 0005. The urine qualitative measurement result of the urine S1 of the sample number 0005 is not less than the threshold value but, in this case, the washing is performed with the standard condition. In such a case, it is considered that the clinically accurate washing is not required.

It goes without saying that the washing control section 19Ab can determine the washing condition based on the type of the biological sample S or based on the type of the biological sample S and the urine qualitative measurement result without considering the relationship with the type of the biological sample S that is measured next.

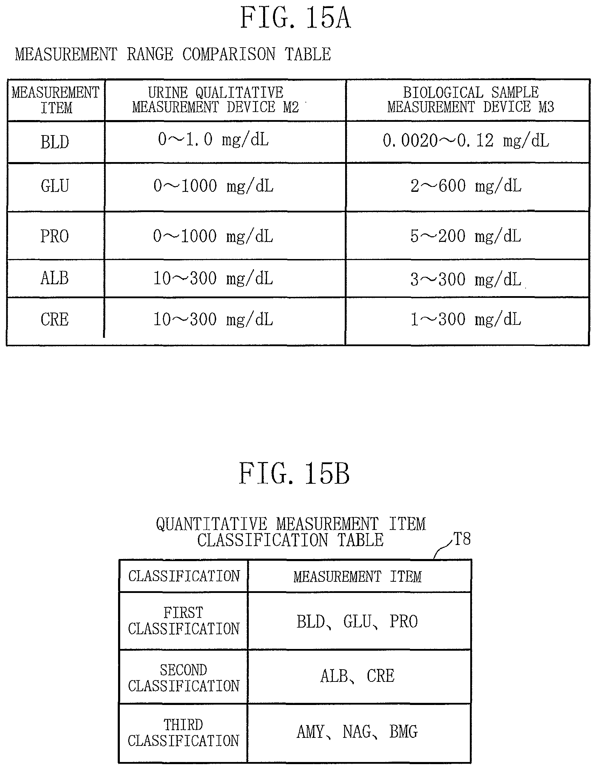

FIG. 15A shows a table in which, with regard to BLD, GLU, PRO, ALB, and CRE as common measurement items, the measurement range of the urine qualitative measurement device M2 is compared with the measurement range of the biological sample measurement device M3.

When attention is paid to BLD in this table, in the urine qualitative measurement device M2, BLD measurement is performed by a method for detecting a peroxidase-like activity of hemoglobin using a test piece including tetramethylbenzidine and cumene hydroperoxide. In contrast to this, in the biological sample measurement device M3, the BLD measurement is performed by latex agglutination turbidimetry that detects agglutination of latex particles by an antigen-antibody reaction. In this case, as is apparent from FIG. 15A, with regard to BLD, the measurement range of the biological sample measurement device M3 is narrower than the measurement range of the urine qualitative measurement device M2. Accordingly, in the case of BLD, in the case where the measurement result higher than the measurement range of the biological sample measurement device M3 is exhibited at the stage of the measurement by the urine qualitative measurement device M2, there is a high possibility that it is not possible to perform accurate measurement in a high-concentration area. This can also occur in the case of GLU and PRO.

Further, with regard to PRO, in the biological sample measurement device M3, the quantitative measurement is performed by using a phenomenon in which, when red bromopyrogallol red-indium complex combines with protein under an acid condition, the maximum absorption wave length shifts to the side of a long wave length and the color is changed to purple. It is well known that, in the case where the quantitative measurement is performed by using this measurement principle, a prozone-like phenomenon can occur. In the case where the quantitative measurement is performed under the condition that the prozone-like phenomenon can occur, there are cases where the measurement result is not displayed as an abnormal value, which leads to a problem.

Therefore, in the case where the measurement item is BLD, GLU or PRO, it is preferable to take measures by referring to the result of the urine qualitative measurement by the urine qualitative measurement device M2 before the quantitative measurement is performed.

On the other hand, as shown in FIG. 15A, with regard to CRE, the measurement range of the urine quantitative measurement is wider than or equal to the measurement range of the urine qualitative measurement. Consequently, in the case where the measurement item is CRE, there is a high possibility that the accurate quantitative measurement result can be acquired even without taking measures in advance. The same applies to ALB.

Based on the foregoing, the measurement item of the biological sample measurement device M3 can be classified as in, e.g., a quantitative measurement item classification table T8 shown in FIG. 15B. That is, the measurement item that is measured also in the urine qualitative measurement device M2 and has the measurement range narrower than that of the urine qualitative measurement is classified as a first classification. Specifically, BLD, GLU, and PRO are classified as the first classification. The measurement item that is measured also in the urine qualitative measurement device M2 and has the measurement range wider than or equal to that of the urine qualitative measurement is classified as a second classification. Specifically, CRE and ALB are classified as the second classification. In addition, the measurement item that is not measured in the urine qualitative measurement device M2 is classified as a third classification. Specifically, AMY, NAG, and BMG are classified as the third classification.

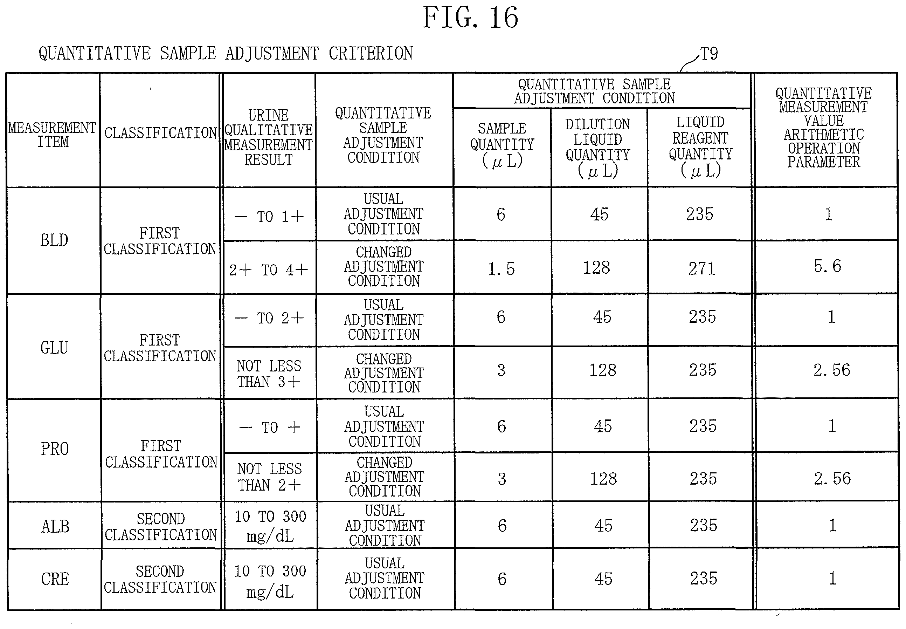

In the case where the measurement item belongs to the first classification, the measurement control section 19Ac determines whether or not a quantitative sample adjustment condition is changed in accordance with the level of the urine qualitative measurement result performed in the urine qualitative measurement device M2. FIG. 16 shows an example of a quantitative sample adjustment criterion including the quantitative sample adjustment condition stored in the quantitative sample adjustment criterion storage section 18Ag. A quantitative sample adjustment criterion T9 shows a threshold value for selection of the urine quantitative sample adjustment condition. This threshold value corresponds to an example of a second threshold value in the present invention. For example, when attention is paid to BLD, the threshold value is present between the qualitative value 1+ and the qualitative value 2+. That is, in the case where the level of the urine qualitative measurement result is not less than the qualitative value 2+ (the semi-quantitative value 0.2), the measurement control section 19Ac determines that the quantitative sample adjustment condition is to be changed. In this case, the measurement control section 19Ac changes the adjustment condition such that the dilution factor of the urine S1 is increased. In contrast to this, in the case where the level of the urine qualitative measurement result is not more than the qualitative value 1+ (the semi-quantitative value 0.06), the measurement control section 19Ac determines that the quantitative sample adjustment condition is not changed. In this case, a usual adjustment condition is selected as the quantitative sample adjustment condition. The same determination is performed on GLU, PRO, ALB, and CRE.

The arithmetic operation parameter storage section 18Ah is a section that stores the arithmetic operation parameter. The arithmetic operation parameter constitutes part of the quantitative sample adjustment criterion T9. In the case where the urine S1 is adjusted with a changed adjustment condition, the arithmetic operation section 19Ad corrects the urine quantitative measurement value such that the urine quantitative measurement value becomes equal to that in the case where the urine S1 is adjusted with the usual adjustment condition. In the biological sample measurement device M3, the common measurement reagent is used irrespective of the type of the biological sample and the level of the urine qualitative measurement result acquired by the urine qualitative measurement device M2, and hence the above correction is required.

The communication sections 17 and 47 are connected to each other via the communication line L, and perform data exchange between the urine qualitative measurement device M2 and the biological sample measurement device M3.

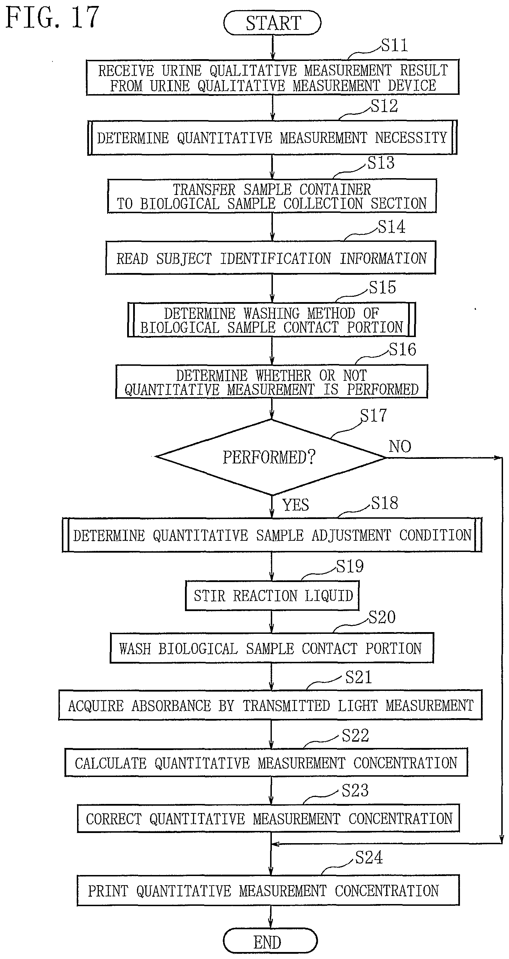

Next, a description will be given of an example of operation process procedures by the control section 19A in the biological sample measurement system MS1 with reference to flowcharts shown in FIGS. 17 to 20 and FIGS. 8 to 16. Note that, herein, the description will be made by taking the BLD measurement of the urine S1 as an example. The following procedures are performed similarly on the other measurement items such as GLU, PRO, and CRE.

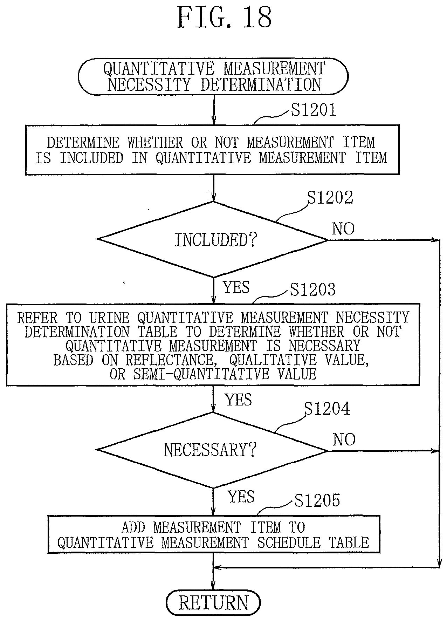

As shown in the flowchart in FIG. 17, when the measurement control section 19Ac receives the qualitative measurement result of BLD of the corresponding urine S1 from the urine qualitative measurement device M2 (S11), the measurement control section 19Ac determines whether or not the quantitative measurement of the urine S1 is necessary (S12). The quantitative measurement necessity determination is performed in accordance with a subroutine shown in FIG. 18.

As shown in the flowchart in FIG. 18, the measurement control section 19Ac refers to the measurement item comparison table T3 to determine whether or not BLD is included in the measurement item of the biological sample measurement device M3 (S1201). When the measurement control section 19Ac determines that BLD is included in the measurement item of the biological sample measurement device M3, the measurement control section 19Ac refers to the urine quantitative measurement necessity determination table T4 to determine whether or not the automatic quantitative measurement is necessary (S1202: YES, S1203). In the case where the urine qualitative measurement result of BLD is not less than the threshold value (the qualitative value ±, the semi-quantitative value 0.03), the measurement control section 19Ac determines that the quantitative measurement is necessary (S1204: YES). The measurement control section 19Ac additionally writes the urine qualitative measurement result into the quantitative measurement schedule table T5 in association with the subject identification information (S1205).

On the other hand, in the case where the measurement control section 19Ac determines that the measurement item is not included in the measurement item of the biological sample measurement device M3, the procedures return to the flowchart in FIG. 17 (S1202: NO). In the case where the urine qualitative measurement result of BLD is not more than the threshold value (the qualitative value -), the measurement control section 19Ac determines that the quantitative measurement is not necessary (S1204: NO). When the above processes are completed, the procedures return to the flowchart in FIG. 17.

Next, as shown in the flowchart in FIG. 17, when the rack 3 that holds the urine container 30A in which the urine S1 is contained moves from the transfer device 2A to the transfer device 2B, the rack 3 is transferred toward the biological sample collection section 27 that is positioned midway in a transfer path indicated by the arrow N2 (S13). While the rack 3 is transferred toward the biological sample collection section 27, the subject identification information recorded in the urine container 30A is read by the ID reading section 11 (S14).

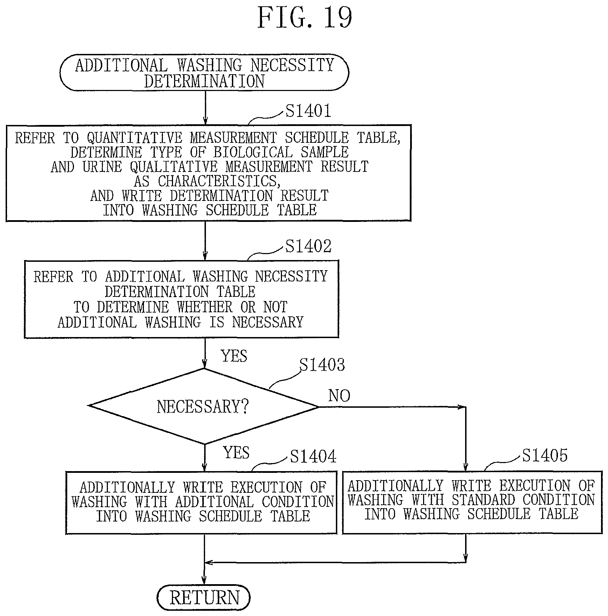

Next, the control section 19A determines the washing method of the biological sample contact portion (S14). The determination of the washing method of the biological sample contact portion is performed in accordance with a subroutine shown in FIG. 19. FIG. 19 shows an example of procedures in which the control section 19A determines the washing method based on the type of the biological sample S and the urine qualitative measurement result acquired by the urine qualitative measurement device M2.

First, the characteristic judgment section 19Aa refers to the quantitative measurement schedule table T5 to write the type of the biological sample S into the washing schedule table T7 as the characteristic. Further, the characteristic judgment section 19Aa determines that the level of the urine qualitative measurement result is not less than the predetermined threshold value or not more than the predetermined threshold value by using the level of the urine qualitative measurement result as the characteristic, and writes the determination result into the washing schedule table T7 (S1401).

Next, the washing control section 19Ab refers to the additional washing necessity determination table T6 to determine whether or not the additional washing of the urine S1 described in the quantitative measurement schedule table T5 is necessary (S1402). In the case where the urine qualitative measurement result of BLD is not less than the threshold value (the qualitative value 2+, the semi-quantitative value 0.2), and the next biological sample S is the feces S2, the washing control section 19Ab determines that the additional washing is necessary (S1403: YES). Next, the washing control section 19Ab records the execution of the washing with the additional condition for each corresponding urine S1 in the washing schedule table T7 (S1404).

On the other hand, in the case where the urine qualitative measurement result of BLD is not more than the threshold value (the qualitative value 1+, the semi-quantitative value 0.06), the washing control section 19Ab determines that the additional washing is not necessary (S1403: NO). Even when the urine qualitative measurement result of BLD is not less than the threshold value (the qualitative value 2+, the semi-quantitative value 0.2), in the case where the next biological sample S is the urine S1, the washing control section 19Ab determines that the additional washing is not necessary (S1403: NO). Next, the washing control section 19Ab records the execution of the washing with the standard condition for each corresponding urine S1 in the washing schedule table T7 (S1405). When the above processes are completed, the procedures return to the flowchart shown in FIG. 17.

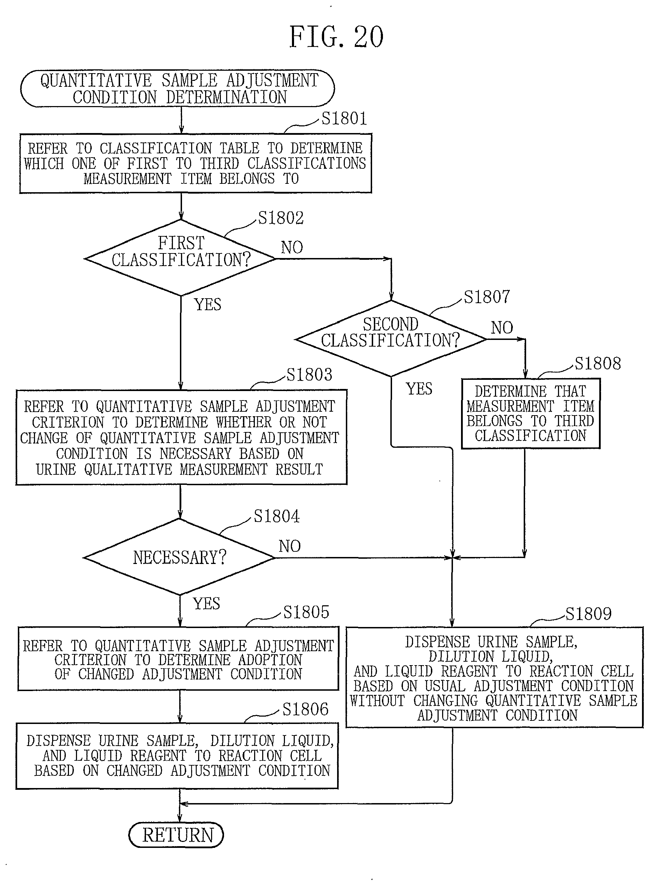

Next, the measurement control section 19Ac refers to the quantitative measurement schedule table T5 based on the subject identification information read by the ID reading section 11 to determine whether or not the quantitative measurement of BLD is scheduled to be performed (S16). In the case where the measurement control section 19Ac determines that the quantitative measurement is scheduled to be performed, the measurement control section 19Ac determines the quantitative measurement sample adjustment condition (S17: YES, S18). The determination of the quantitative sample adjustment condition is performed in accordance with a subroutine shown in FIG. 20.

As shown in the flowchart in FIG. 20, the measurement control section 19Ac refers to the quantitative measurement item classification table T8 to determine which one of the first to third classifications BLD belongs to (S1801). Judging from the quantitative measurement item classification table T8, BLD belongs to the first classification, and hence the measurement control section 19Ac refers to the quantitative sample adjustment criterion T9 to determine whether or not the quantitative sample adjustment condition is changed based on the level of the urine qualitative measurement result (S1802: YES, S1803).

The measurement control section 19Ac refers to the quantitative sample adjustment criterion T9 and, in the case where the level of the urine qualitative measurement result of BLD is not less than the threshold value (the qualitative value 2+, the semi-quantitative value 0.2), the measurement control section 19Ac determines that the changed adjustment condition is adopted (S1804 : YES, S1805) . Next, the measurement control section 19Ac causes the sample collection nozzle 10A to dispense the urine S1, the dilution liquid, and the liquid reagent to the reaction cell 13b based on the changed adjustment condition (S1806) . With this, the dilution factor of the urine S1 is changed from the dilution factor of the usual adjustment condition. In contrast to this, in the case where the level of the urine qualitative measurement result of BLD is not more than the threshold value (the qualitative value 1+, the semi-quantitative value 0.06), the sample collection nozzle 10A dispenses the urine S1, the dilution liquid, and the liquid reagent based on the usual adjustment condition (S1804: NO, S1809).

Note that, in the case where the measurement item belongs to the second or third classification (S1802: NO), the dispensing based on the usual adjustment condition is also performed (S1807 to S1809) . When the above processes are completed, the procedures return to the flowchart shown in FIG. 17.

Next, as shown in the flowchart in FIG. 17, the cell stirring section 10C performs the stirring of the reaction liquid in the reaction cell (S19). Next, the washing section 12 refers to the washing schedule table T7 to wash the sample collection nozzle 10A and the stirring blade 10Ca that have contacted the urine S1. The washing section 12 is capable of starting the washing before the measurement of the urine S1 is completed. For example, the washing of the sample collection nozzle 10A is started at the point of time when the washing method of the urine S1 is determined, and the dispensing to the reaction cell 13b is completed. In addition, the washing of the stirring blade 10Ca is started at the point of time when the washing method of the urine S1 is determined and the stirring of the reaction liquid in the reaction cell 13b is completed. That is, the washing control section 19Ab is capable of starting the washing from the biological sample contact portion of which passage of the reaction liquid is completed sequentially. It goes without saying that the washing may be started after the passage of the reaction liquid on the biological sample contact portion is all completed.

Next, the measurement section 13 performs transmitted light measurement to acquire the absorbance (S21). The arithmetic operation section 19Ad applies the absorbance to the calibration curve stored in the storage section to calculate the quantitative measurement concentration of BLD (S22). Further, the arithmetic operation section 19Ad corrects the quantitative measurement concentration by using the arithmetic operation parameter recorded in the quantitative sample adjustment criterion T9 (S23). Next, the printer 14 prints the corrected quantitative measurement concentration in association with the subject identification information (S24).

According to the present embodiment, as the characteristic of the urine S1, the urine qualitative measurement result acquired by the urine qualitative measurement device M2 is adopted. The urine qualitative measurement result is information that can be acquired in the biological sample measurement device M3 without performing the measurement on the urine S1. Accordingly, the washing control section 19Ab can early determine whether or not the additional washing of the biological sample contact portion is necessary. In addition, the washing control section 19Ab can select the proper washing condition in accordance with the level of the urine qualitative measurement result instead of using the uniform washing condition. With this, it is possible to achieve acceleration of the measurement while maintaining accuracy in the measurement by the measurement section 13.

According to the present embodiment, as the characteristic of the urine S1, the urine qualitative measurement result acquired by the urine qualitative measurement device M2 is adopted in addition to the type of the biological sample S. Accordingly, the washing control section 19Ab can set the washing condition of the biological sample contact portion minutely and properly. With this, it is possible to achieve acceleration of the measurement while maintaining accuracy in the measurement by the measurement section 13.

According to the present embodiment, as the characteristic of the urine S1, the urine qualitative measurement result acquired by the urine qualitative measurement device M2 is adopted. The characteristic judgment section 19Aa of the biological sample measurement device M3 uses the information when the characteristic judgment section 19Aa judges the characteristic of the urine S1. Accordingly, it is not necessary to add a new detection device or the like to the biological sample measurement device M3 in order to obtain the characteristic. With this, it is possible to prevent an increase in the manufacturing cost of the biological sample measurement system MS1.

According to the present embodiment, when the measurement item in the biological sample measurement device M3 is the occult blood, in the case where the characteristic judgment section 19Aa determines that the level of the urine qualitative measurement result is not less than the preset first threshold value, the washing control section 19Ab causes the washing section 12 to wash the biological sample contact portion with the condition in which the washing liquid quantity or the washing time is increased. Since the washing liquid quantity or the washing time is increased as the washing condition, it is not necessary to add a new washing device or the like. With this, it is possible to prevent an increase in the manufacturing cost of the biological sample measurement system MS1.

According to the present embodiment, in the case where the urine qualitative measurement result of the occult blood is not less than the preset second threshold value, the measurement control section 19Ac causes the measurement section 13 to automatically perform the urine occult blood quantitative measurement. Accordingly, it is possible to prevent execution of unnecessary measurement in the biological sample measurement device M3. With this, it is possible to achieve acceleration of the measurement and prevent an increase in measurement cost in the biological sample measurement system MS1.

According to the present embodiment, the measurement section 13 is configured to use the common measurement reagent in the urine occult blood quantitative measurement in the biological sample measurement device M3 irrespective of the urine qualitative measurement result of the occult blood. The measurement control section 19Ac controls the measurement method of the occult blood quantitative measurement in the measurement section 13 based on the level of the urine qualitative measurement result of the occult blood in the urine qualitative measurement device M2. Accordingly, it is possible to reduce the frequency of re-measurement in the measurement section 13. With this, it is possible to achieve acceleration of the measurement and a reduction in measurement cost.

According to the present embodiment, the measurement items of the biological sample measurement device M3 are classified as the first to third classifications in advance. In the case where the measurement item belongs to the first classification, the measurement control section 19Ac determines whether or not the quantitative sample adjustment condition is changed based on the level of the urine qualitative measurement result. With this, it is not necessary to determine the necessity to change the quantitative sample adjustment condition on all of the measurement items, and hence it is possible to reduce the burden of the measurement control section 19Ac.

The present invention is not limited to the contents of the embodiments described above. The specific configurations of the individual sections of the biological sample measurement device and the biological sample measurement system according to the present invention can be modified in design in various ways. In addition, the specific configurations of the individual steps of the biological sample measurement method of the sample according to the present invention can be modified in various ways.

As described in the first example, the sample container 30 can also be configured such that the type of the biological sample S is recorded together with the subject identification information. In this case, the ID reading section 11 is configured to be capable of reading the type of the biological sample S. The characteristic judgment section 19a determines the type of the biological sample S read by the ID reading section 11 as the characteristic of the biological sample S. With this configuration, the type of the biological sample S is directly recorded in the sample container 30 in which the biological sample S is contained, and hence the judgment of the characteristic by the characteristic judgment section 19 Aa becomes more reliable. With this, the washing control section 19Ab can reliably select the washing condition of the biological sample contact portion.

In the first example, the sample container 30 can be set such that its height and shape vary by the type of the biological sample S, and the sample container 30 can be identified by a shape detection sensor. In this case, the characteristic judgment section 19a determines the type of the biological sample S as the characteristic by detecting a difference in the shape of the sample container 30. Accordingly, it is possible to early determine whether or not the change of the washing condition is necessary. With this, it is possible to achieve acceleration of the measurement and a reduction in measurement cost.

In the first embodiment, in the case where the urine qualitative measurement result is not less than the second threshold value, it is also possible to cancel the occult blood measurement instead of changing the sample adjustment condition of the urine S1. With this configuration, it becomes possible to prevent the execution of the unnecessary measurement. With this, it is possible to achieve acceleration of the measurement and prevent an increase in measurement cost in the biological sample measurement device M3.
- MS1: biological sample measurement system
- M1, M3: biological sample measurement device
- M2: urine qualitative measurement device (external equipment)
- S: biological sample
- S1: urine
- S2: feces
- 11, 41: reading section
- 12: washing section
- 13: measurement section
- 19a, 19Aa: characteristic judgment section
- 19b, 19Ab: washing control section
- 19Ac: measurement control section
- 30: sample container
- 30A: urine container (sample container)
- 30B: feces container (sample container)

## Claims

1. A biological sample measurement system comprising: a biological sample measurement device (M3) and external equipment (M2) connected to the biological sample measurement device, wherein
the biological sample measurement device (M3) comprises:
a measurement section (13) for performing measurement of a biological sample;
a washing section (12) for washing a biological sample contact portion that contacts the biological sample during the measurement;
a characteristic judgment section (19Aa) for judging a characteristic of the biological sample before the measurement is completed; and
a washing control section (19Ab) for determining a washing method by the washing section (12) based on the characteristic judged by the characteristic judgment section (19Aa),
the characteristic judgment section (19Aa) is configured to acquire the characteristic from the external equipment (M2),
the external equipment (M2) is a urine qualitative measurement device that is configured to perform urine qualitative measurement on the urine by using a urine test paper to acquire a urine qualitative measurement result of a predetermined measurement item,
the biological sample is urine,
the biological sample measurement device (M3) is configured to perform measurement on the urine on which the urine qualitative measurement has been performed in the urine qualitative measurement device (M2),
the characteristic judgment section (19Aa) is configured to receive the urine qualitative measurement result from the urine qualitative measurement device (M2) and to determine a level of the urine qualitative measurement result as the characteristic,
the level of the urine qualitative measurement result is a level of urine occult blood, and
the washing control section (19Ab) is configured to determine a washing method of the biological sample contact portion contacted by the urine used for the urine qualitative measurement by the washing section (12) based on the level.

2. The biological sample measurement system according to claim 1, wherein
the measurement section (13) is further configured to perform measurement of a plurality of types of biological samples sequentially, urine being one of the plurality of types.

3. The biological sample measurement system according to claim 2, wherein
the characteristic judgment section (19Aa) is further configured to determine the type of the biological sample as a further characteristic, and
the washing control section (19Ab) is further configured to determine the washing method based on the type.

4. The biological sample measurement system according to claim 3, wherein
the type is one of urine and feces, and
the characteristic judgment section (19Aa) is further configured to determine whether the type is the urine or the feces as the further characteristic.

5. The biological sample measurement system according to claim 4, wherein
in a case where the measurement section (13) performs the measurement of the feces subsequently to the measurement of the urine, the washing control section (19Ab) is further configured to cause the washing section (12) to wash the biological sample contact portion contacted by the urine under a condition in which a washing liquid quantity or washing time is increased.

6. The biological sample measurement system according to claim 4, further comprising a measurement control section (19Ac) for controlling a measurement method in the measurement section (13), wherein
the measurement section (13) is configured to measure an occult blood, and
the measurement control section (19Ac) is configured to use a common measurement reagent irrespective of the type.

7. The biological sample measurement system according to any one of claims 2 to 6, wherein
the biological sample is contained in a sample container on which subject identification information is recorded,
the biological sample measurement device (M3) further comprises:
a reading section (11) for reading the subject identification information; and
a storage section (18A) for storing in advance pair information in which the subject identification information is paired with the type associated with the subject identification information, and
the characteristic judgment section (19Aa) is further configured to refer to the pair information based on the subject identification information read by the reading section to determine the type as the further characteristic.

8. The biological sample measurement system according to claim 7, wherein
the storage section (18A) is configured to store a measurement schedule table in which a schedule of the measurement of the biological sample is described,
the measurement schedule table includes the pair information, and
in a case where, subsequently to the measurement of a first biological sample of a predetermined type, the measurement of a second biological sample of a predetermined type different from the first biological sample is scheduled to be performed in the measurement schedule table, the washing control section (19Ab) is further configured to determine the washing method of the biological sample contact portion contacted by the first biological sample in accordance with a combination of the first biological sample and the second biological sample.

9. The biological sample measurement system according to any one of claims 2 to 8, wherein
the biological sample is contained in a sample container on which subject identification information is recorded,
the biological sample measurement device (M3) further comprises a reading section (11) for reading the subject identification information,
the type is also recorded in the sample container,
the reading section (11) is capable of reading the type, and
the characteristic judgment section (19Aa) is further configured to determine the type read by the reading section as the characteristic.

10. The biological sample measurement system according to claim 9, wherein
in a case where, subsequently to the measurement of a first biological sample of a predetermined type, the measurement of a second biological sample of a predetermined type different from the first biological sample is scheduled to be performed, the washing control section (19Ab) is further configured to determine the washing method of the biological sample contact portion contacted by the first biological sample in accordance with a combination of the first biological sample and the second biological sample.

11. A biological sample measurement method, comprising:
a measurement step (S12) of performing measurement of a biological sample;
a washing step (S20) of washing a biological sample contact portion, the biological sample contact portion contacting the biological sample during the measurement;
a urine qualitative measurement step of performing urine qualitative measurement on the urine by using a urine test paper to acquire a urine qualitative measurement result of a predetermined measurement item before the measurement step is completed;
a characteristic judgment step (S1401) of judging a characteristic of the biological sample before the measurement is completed; and
a determination step (S15) of determining a washing method of the biological sample contact portion based on the characteristic judged in the judgment step; wherein
the biological sample is urine,
in the measurement step (S12), the measurement is performed on the urine on which the urine qualitative measurement has been performed in the urine qualitative measurement step,
in the characteristic judgment step (S1401), a level of the urine qualitative measurement result is judged as the characteristic,
the level of the urine qualitative measurement result is a level of urine occult blood, and
in the determination step (S15), a washing method of the biological sample contact portion contacted by the urine used for the urine qualitative measurement is determined based on the level of the urine qualitative measurement result.

## Patentansprüche

1. Ein biologisches Probenmesssystem, das Folgendes beinhaltet: eine biologische Probenmessvorrichtung (M3) und externe Ausrüstung (M2), die mit der biologischen Probenmessvorrichtung verbunden ist, wobei
die biologische Probenmessvorrichtung (M3) Folgendes beinhaltet:
einen Messabschnitt (13) zur Durchführung der Messung einer biologischen Probe;
einen Waschabschnitt (12) zum Waschen eines biologischen Probenkontaktbereichs, der während der Messung mit der biologischen Probe in Kontakt kommt;
einen Merkmalbeurteilungsabschnitt (19Aa) zur Beurteilung eines Merkmals der biologischen Probe, bevor die Messung abgeschlossen ist; und
einen Waschkontrollabschnitt (19Ab) zur Bestimmung eines Waschverfahrens durch den Waschabschnitt (12) auf der Grundlage des von dem Merkmalbeurteilungsabschnitt (19Aa) beurteilten Merkmals,
der Merkmalbeurteilungsabschnitt (19Aa) dazu konfiguriert ist, das Merkmal von der externen Ausrüstung (M2) zu akquirieren,
die externe Ausrüstung (M2) eine Vorrichtung zur qualitativen Urinmessung ist, die dazu konfiguriert ist, eine qualitative Urinmessung an dem Urin unter Verwendung eines Urintestpapiers durchzuführen, um ein qualitatives Urinmessergebnis eines vorbestimmten Messartikels zu akquirieren,
die biologische Probe Urin ist,
die biologische Probenmessvorrichtung (M3) dazu konfiguriert ist, eine Messung an dem Urin durchzuführen, an dem die qualitative Urinmessung in der qualitativen Urinmessvorrichtung (M2) durchgeführt wurde,
der Merkmalbeurteilungsabschnitt (19Aa) dazu konfiguriert ist, das qualitative Urinmessergebnis von der qualitativen Urinmessvorrichtung (M2) zu empfangen und einen Spiegel des qualitativen Urinmessergebnisses als das Merkmal zu bestimmen,
der Spiegel des qualitativen Urinmessergebnisses ein Spiegel von okkultem Blut im Urin ist und
der Waschkontrollabschnitt (19Ab) dazu konfiguriert ist, ein Waschverfahren des biologischen Probenkontaktbereichs, der mit dem Urin in Kontakt kommt, der für die qualitative Urinmessung durch den Waschabschnitt (12) auf der Grundlage des Spiegels verwendet werden soll, zu bestimmen.

2. Biologisches Probenmesssystem nach Anspruch 1, wobei
der Messabschnitt (13) ferner dazu konfiguriert ist, die Messung einer Vielzahl von Typen biologischer Proben nacheinander durchzuführen, wobei Urin einer der Vielzahl von Typen ist.

3. Biologisches Probenmesssystem nach Anspruch 2, wobei
der Merkmalbeurteilungsabschnitt (19Aa) ferner dazu konfiguriert ist, den Typ der biologischen Probe als ein weiteres Merkmal zu bestimmen, und der
Waschkontrollabschnitt (19Ab) ferner dazu konfiguriert ist, das Waschverfahren auf der Grundlage des Typs zu bestimmen.

4. Biologisches Probenmesssystem nach Anspruch 3, wobei
der Typ einer von Urin und Stuhl ist und
der Merkmalbeurteilungsabschnitt (19Aa) ferner dazu konfiguriert ist zu bestimmen, ob es sich bei dem Typ um Urin oder Stuhl handelt, als das weitere Merkmal.

5. Biologisches Probenmesssystem nach Anspruch 4, wobei
in einem Fall, in dem der Messabschnitt (13) die Messung des Stuhls nach der Messung des Urins durchführt, der Waschkontrollabschnitt (19Ab) ferner dazu konfiguriert ist, zu bewirken, dass der Waschabschnitt (12) den biologischen Probenkontaktbereich, der mit dem Urin in Kontakt kommt, zu waschen, unter einer Bedingung, in der eine Waschflüssigkeitsmenge oder Waschzeit erhöht wird.

6. Biologisches Probenmesssystem nach Anspruch 4, das ferner einen Messkontrollabschnitt (19Ac) zum Kontrollieren eines Messverfahrens in dem Messabschnitt (13) beinhaltet, wobei
der Messabschnitt (13) zur Messung von okkultem Blut konfiguriert ist und
der Messkontrollabschnitt (19Ac) zur Verwendung eines gemeinsamen Messreagenzes, unabhängig von dem Typ, konfiguriert ist.

7. Biologisches Probenmesssystem nach einem der Ansprüche 2 bis 6, wobei
die biologische Probe in einem Probenbehälter enthalten ist, auf dem Patientenidentifikationsinformationen aufgezeichnet sind,
die biologische Probenmessvorrichtung (M3) ferner Folgendes beinhaltet:
einen Leseabschnitt (11) zum Lesen der Patientenidentifikationsinformationen; und
einen Speicherabschnitt (18A) zum Speichern Speicherung von Paarinformationen im Voraus, in denen die Patientenidentifikationsinformationen mit dem Typ gepaart sind, der mit den Patientenidentifikationsinformationen assoziiert ist, und
der Merkmalbeurteilungsabschnitt (19Aa) ferner zur Bezugnahme auf die Paarinformationen konfiguriert ist, die auf den von dem Leseabschnitt gelesenen Patientenidentifikationsinformationen basieren, um den Typ des weiteren Merkmals zu bestimmen.

8. Biologisches Probenmesssystem nach Anspruch 7, wobei
der Speicherabschnitt (18A) dazu konfiguriert ist, eine Messzeitplantabelle zu speichern, in der ein Zeitplan der Messung der biologischen Probe beschrieben ist,
die Messzeitplantabelle die Paarinformationen umfasst, und
in einem Fall, in dem nach der Messung einer ersten biologischen Probe eines vorbestimmten Typs die Durchführung einer Messung einer zweiten biologischen Probe eines vorbestimmten Typs, der von der ersten biologischen Probe verschieden ist, in der Messzeitplantabelle geplant ist, der Waschkontrollabschnitt (19Ab) ferner dazu konfiguriert ist, das Waschverfahren des biologischen Probenkontaktbereichs, der mit der ersten biologischen Probe in Kontakt kommt, gemäß einer Kombination der ersten biologischen Probe und der zweiten biologischen Probe zu bestimmen.

9. Biologisches Probenmesssystem nach einem der Ansprüche 2 bis 8, wobei
die biologische Probe in einem Probenbehälter enthalten ist, auf dem Patientenidentifikationsinformationen aufgezeichnet sind,
die biologische Probenmessvorrichtung (M3) ferner einen Leseabschnitt (11) zum Lesen der Patientenidentifikationsinformationen beinhaltet,
der Typ ebenfalls in dem Probenbehälter aufgezeichnet ist,
der Leseabschnitt (11) in der Lage ist, den Typ zu lesen, und
der Merkmalbeurteilungsabschnitt (19Aa) ferner dazu konfiguriert ist, den von dem Leseabschnitt gelesenen Typ als das Merkmal zu bestimmen.

10. Biologisches Probenmesssystem nach Anspruch 9, wobei
in einem Fall, in dem nach der Messung einer ersten biologischen Probe eines vorbestimmten Typs die Durchführung der Messung einer zweiten biologischen Probe eines vorbestimmten Typs, der von der ersten biologischen Probe verschieden ist, geplant ist, der Waschkontrollabschnitt (19Ab) ferner dazu konfiguriert ist, das Waschverfahren des biologischen Probenkontaktbereichs, der mit der ersten biologischen Probe in Kontakt kommt, gemäß einer Kombination der ersten biologischen Probe und der zweiten biologischen Probe zu bestimmen.

11. Ein biologisches Probenmessverfahren, das Folgendes beinhaltet:
einen Messschritt (S12) des Durchführens der Messung einer biologischen Probe;
einen Waschschritt (S20) des Waschens eines biologischen Probenkontaktbereichs, wobei der biologische Probenkontaktbereich während der Messung mit der biologischen Probe in Kontakt kommt;
einen qualitativen Urinmessschritt des Durchführens einer qualitativen Urinmessung an dem Urin unter Verwendung eines Urintestpapiers, um ein qualitatives Urinmessergebnis eines vorbestimmten Messartikels zu akquirieren, bevor der Messschritt abgeschlossen ist;
einen Merkmalbeurteilungsschritt (S1401) des Beurteilens eines Merkmals der biologischen Probe, bevor die Messung abgeschlossen ist; und
einen Bestimmungsschritt (S15) des Bestimmens eines Waschverfahrens des biologischen Probenkontaktbereichs auf der Grundlage des in dem Beurteilungsschritts beurteilten Merkmals; wobei
die biologische Probe Urin ist,
in dem Messschritt (S12) die Messung an dem Urin durchgeführt wird, an dem die qualitative Urinmessung in dem qualitativen Urinmessschritt durchgeführt wurde,
in dem Merkmalsbeurteilungsschritt (S1401) ein Spiegel des qualitativen Urinmessergebnisses als das Merkmal beurteilt wird,
der Spiegel des qualitativen Urinmessergebnisses ein Spiegel von okkultem Blut im Urin ist und
in dem Bestimmungsschritt (S15) ein Waschverfahren des biologischen Probenkontaktbereichs, der mit dem Urin in Kontakt kommt, der für die qualitative Urinmessung verwendet wird, auf der Grundlage des Spiegels des qualitativen Urinmessergebnisses bestimmt wird.

## Revendications

1. Système de mesure d'échantillon biologique, comprenant : un dispositif de mesure d'échantillon biologique (M3) et un équipement externe (M2) connecté au dispositif de mesure d'échantillon biologique, dans lequel :
le dispositif de mesure d'échantillon biologique (M3) comprend :
une section de mesure (13), pour réaliser la mesure d'un échantillon biologique ;
une section de lavage (12), pour le lavage d'une partie en contact avec l'échantillon biologique qui entre en contact avec l'échantillon biologique durant la mesure ;
une section d'évaluation de caractéristique (19Aa), pour l'évaluation d'une caractéristique de l'échantillon biologique avant que la mesure ne soit terminée ; et
une section de commande de lavage (19Ab), pour la détermination d'une méthode de lavage par la section de lavage (12), à partir de la caractéristique évaluée par la section d'évaluation de caractéristique (19Aa),
la section d'évaluation de caractéristique (19Aa) est configurée pour obtenir la caractéristique à partir de l'équipement externe (M2),
l'équipement externe (M2) est un appareil de mesure qualitative d'urine qui est configuré pour réaliser une mesure qualitative d'urine sur l'urine, en utilisant un papier d'analyse d'urine, pour obtenir un résultat de mesure qualitative d'urine correspondant à un élément de mesure prédéterminé,
l'échantillon biologique est de l'urine,
le dispositif de mesure d'échantillon biologique (M3) est configuré pour réaliser une mesure sur l'urine sur laquelle la mesure qualitative d'urine a été réalisée dans le dispositif de mesure qualitative d'urine (M2),
la section d'évaluation de caractéristique (19Aa) est configurée pour recevoir le résultat de mesure qualitative d'urine à partir du dispositif de mesure qualitative d'urine (M2) et pour déterminer un niveau du résultat de mesure qualitative d'urine, en tant que caractéristique,
le niveau du résultat de mesure qualitative d'urine est un niveau de sang occulte dans l'urine, et
la section de commande de lavage (19Ab) est configurée pour déterminer une méthode de lavage, par la section de lavage (12), de la partie en contact avec l'échantillon biologique avec laquelle l'urine utilisée pour la mesure qualitative d'urine entre en contact, en fonction du niveau.

2. Système de mesure d'échantillon biologique selon la revendication 1, dans lequel :
la section de mesure (13) est configurée en outre pour réaliser séquentiellement la mesure d'une pluralité de types d'échantillons biologiques, l'urine étant l'un des types de la pluralité de types.

3. Système de mesure d'échantillon biologique selon la revendication 2, dans lequel :
la section d'évaluation de caractéristique (19Aa) est configurée en outre pour déterminer le type de l'échantillon biologique, en tant que caractéristique supplémentaire, et
la section de commande de lavage (19Ab) est configurée en outre pour déterminer la méthode de lavage à partir du type.

4. Système de mesure d'échantillon biologique selon la revendication 3, dans lequel :
le type est l'un de l'urine et des fèces, et
la section d'évaluation de caractéristique (19Aa) est configurée en outre pour déterminer si le type est l'urine ou les fèces, en tant que caractéristique supplémentaire.

5. Système de mesure d'échantillon biologique selon la revendication 4, dans lequel :
dans un cas où la section de mesure (13) réalise la mesure des fèces après la mesure de l'urine, la section de commande de lavage (19Ab) est configurée en outre pour faire en sorte que la section de lavage (12) lave la partie en contact avec l'échantillon biologique avec laquelle l'urine entre en contact, sous une condition dans laquelle une quantité de liquide de lavage ou un temps de lavage est augmenté(e).

6. Système de mesure d'échantillon biologique selon la revendication 4, comprenant en outre une section de commande de mesure (19Ac) servant à contrôler une méthode de mesure dans la section de mesure (13), dans lequel :
la section de mesure (13) est configurée pour mesurer un sang occulte, et
la section de commande de mesure (19Ac) est configurée pour utiliser un réactif de mesure commun, quel que soit le type.

7. Système de mesure d'échantillon biologique selon l'une quelconque des revendications 2 à 6, dans lequel :
l'échantillon biologique est contenu dans un récipient d'échantillon sur lequel des informations d'identification du sujet sont notées,
le dispositif de mesure d'échantillon biologique (M3) comprend en outre :
une section de lecture (11), pour la lecture des informations d'identification du sujet ; et
une section d'enregistrement (18A), pour l'enregistrement à l'avance d'informations appariées, dans laquelle les informations d'identification du sujet sont appariées avec le type associé aux informations d'identification du sujet, et
la section d'évaluation de caractéristique (19Aa) est configurée en outre pour se référer aux informations appariées en se basant sur les informations d'identification du sujet lues par la section de lecture pour déterminer le type, en tant que caractéristique supplémentaire.

8. Système de mesure d'échantillon biologique selon la revendication 7, dans lequel :
la section d'enregistrement (18A) est configurée pour l'enregistrement d'un tableau de planification de mesure, dans lequel une planification de la mesure de l'échantillon biologique est décrite,
le tableau de planification de mesure comprend les informations appariées, et
dans un cas où, après la mesure d'un premier échantillon biologique d'un type prédéterminé, la réalisation d'une mesure d'un deuxième échantillon biologique d'un type prédéterminé différent du premier échantillon biologique est prévue dans le tableau de planification de mesure, la section de commande de lavage (19Ab) est configurée en outre pour déterminer la méthode de lavage de la partie en contact avec l'échantillon biologique, avec laquelle le premier échantillon biologique entre en contact, selon une combinaison du premier échantillon biologique et du deuxième échantillon biologique.

9. Système de mesure d'échantillon biologique selon l'une quelconque des revendications 2 à 8, dans lequel :
l'échantillon biologique est contenu dans un récipient d'échantillon sur lequel des informations d'identification du sujet sont notées,
le dispositif de mesure d'échantillon biologique (M3) comprend en outre une section de lecture (11), pour la lecture des informations d'identification du sujet,
le type est également enregistré dans le récipient d'échantillon,
la section de lecture (11) est capable de lire le type, et
la section d'évaluation de caractéristique (19Aa) est configurée en outre pour déterminer le type lu par la section de lecture, en tant que caractéristique.

10. Système de mesure d'échantillon biologique selon la revendication 9, dans lequel :
dans un cas où, après la mesure d'un premier échantillon biologique d'un type prédéterminé, la réalisation d'une mesure d'un deuxième échantillon biologique d'un type prédéterminé différent du premier échantillon biologique est prévue, la section de commande de lavage (19Ab) est configurée en outre pour déterminer la méthode de lavage de la partie en contact avec l'échantillon biologique, avec laquelle le premier échantillon biologique entre en contact, selon une combinaison du premier échantillon biologique et du deuxième échantillon biologique.

11. Procédé de mesure d'échantillon biologique, comprenant :
une étape de mesure (S12), consistant à mesurer un échantillon biologique ;
une étape de lavage (S20), consistant à laver une partie en contact avec l'échantillon biologique, la partie en contact avec l'échantillon biologique entrant en contact avec l'échantillon biologique durant la mesure ;
une étape de mesure qualitative d'urine, consistant à réaliser une mesure qualitative d'urine sur l'urine, en utilisant un papier d'analyse d'urine, pour obtenir un résultat de mesure qualitative d'urine correspondant à un élément de mesure prédéterminé, avant que l'étape de mesure ne soit terminée ;
une étape d'évaluation de caractéristique (S1401), consistant à évaluer une caractéristique de l'échantillon biologique avant que la mesure ne soit terminée ; et
une étape de détermination (S15), consistant à déterminer une méthode de lavage de la partie en contact avec l'échantillon biologique, à partir de la caractéristique évaluée dans l'étape d'évaluation ; dans lequel
l'échantillon biologique est l'urine,
dans l'étape de mesure (S12), la mesure est réalisée sur l'urine sur laquelle la mesure qualitative d'urine a été réalisée dans l'étape de mesure qualitative d'urine,
dans l'étape d'évaluation de caractéristique (S1401), un niveau du résultat de mesure qualitative d'urine est évalué, en tant que caractéristique,
le niveau du résultat de mesure qualitative d'urine est un niveau de sang occulte dans l'urine, et
dans l'étape de détermination (S15), une méthode de lavage de la partie en contact avec l'échantillon biologique avec laquelle l'urine utilisée pour la mesure qualitative d'urine entre en contact, est déterminée en fonction du niveau du résultat de mesure qualitative d'urine.
